(19) Europäisches Patentamt / European Patent Office / Office européen des brevets

(11) EP 3 897 146 B1

(12) # FASCICULE DE BREVET EUROPEEN

(45) Date de publication et mention de la délivrance du brevet:
**21.05.2025 Bulletin 2025/21**

(21) Numéro de dépôt: **19832335.4**

(22) Date de dépôt: **17.12.2019**

(51) Classification Internationale des Brevets (IPC):
***A01N 35/02*** (2006.01) ***A61K 8/35*** (2006.01)
***A01P 1/00*** (2006.01)

(52) Classification Coopérative des Brevets (CPC):
(C-Sets disponibles)
**A61Q 19/00; A01N 35/02; A61K 8/35;**
A61K 2800/10; A61K 2800/524; A61K 2800/5922
(Cont.)

(86) Numéro de dépôt international:
**PCT/EP2019/085797**

(87) Numéro de publication internationale:
**WO 2020/127383 (25.06.2020 Gazette 2020/26)**

(54) **MÉLANGE ANTIMICROBIEN CONTENANT DU 4-(3-ÉTHOXY-4-HYDROXYPHÉNYL) BUTAN-2-ONE ET LE 4-HYDROXYACÉTOPHENONE, ET COMPOSITION LE CONTENANT**

ANTIMIKROBIELLE MISCHUNG MIT 4-(3-ETHOXY-4-HYDROXYPHENYL)-BUTAN-2-ON UND 4-HYDROXYACETOPHENON SOWIE ZUSAMMENSETZUNG DAMIT

ANTIMICROBIAL MIXTURE CONTAINING 4-(3-ETHOXY-4-HYDROXYPHENYL) BUTAN-2-ONE, AND 4-HYDROXYACETOPHENONE, AND COMPOSITION CONTAINING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.12.2018 FR 1873695**
**03.12.2019 FR 1913639**

(43) Date de publication de la demande:
**27.10.2021 Bulletin 2021/43**

(73) Titulaire: **L'OREAL**
**75008 Paris (FR)**

(72) Inventeurs:
- **EYRAUD, Sonia**
**94152 CHEVILLY LARUE (FR)**
- **GALVAN, Julien**
**94152 CHEVILLY LARUE (FR)**
- **BOSSANT, Isabelle**
**94152 CHEVILLY LARUE (FR)**

(74) Mandataire: **L'Oreal**
**Service D.I.P.I.**
**9, rue Pierre Dreyfus**
**92110 Clichy (FR)**

(56) Documents cités:
**WO-A1-2011/039445 US-A1- 2017 204 351**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 897 146 B1

(52) Classification Coopérative des Brevets (CPC):
(Cont.)

C-Sets
**A01N 35/02, A01N 35/04**

## Description

La présente invention a pour objet un mélange antimicrobien de i) 4-(3-éthoxy-4-hydroxyphényl)butan-2-one et de ii) 4-hydroxyacetophénone ainsi que leurs sels de bases, leurs solvates tels que les hydrates, ainsi qu'une composition cosmétique, pharmaceutique ou alimentaire contenant i) et ii).

## Domaine technique

Les microorganismes peuvent survivre et se propager dans les produits cosmétiques, pharmaceutiques, et alimentaires sans conservateurs. Des conservateurs sont régulièrement ajoutés à toutes les préparations industrielles destinées à être stockées ou conservées pour éviter la croissance microbienne dans le temps.

La contamination microbienne lors de la fabrication de produit industriel est courante, même lorsque des ingrédients de départ mis dans ledit produit sont « propres » i.e. sans la présence de microorganismes contaminants. L'eau par exemple qui est omniprésente dans la plupart des produits cosmétiques, pharmaceutiques ou alimentaires, doit être exempte de microorganismes contaminants. Tous les autres ingrédients doivent également faire l'objet d'un dépistage de la présence de microorganismes contaminants. La propreté pendant la fabrication de ces produits industriels, le traitement des contenus et le remplissage des contenants doit être scrupuleusement surveillé. Malgré ces précautions, l'intégrité microbienne des produits peut nécessiter la présence d'un ou plusieurs agents de conservation compatibles avec le produit et la stabilité de la composition. Les produits ne doivent ni permettre la croissance, ni la viabilité des microorganismes contaminants. Même si une production contraignante en milieu stérile peut être réalisable industriellement, le maintien de la stérilité au cours de l'utilisation reste problématique, car les doigts, les applicateurs cosmétiques et même l'air ambiant ne sont pas stériles. Des conservateurs sont donc nécessaires pour réduire la contamination de microorganismes par les consommateurs lors d'utilisations normales. En règle générale, les microorganismes pathogènes doivent être absents de tous les produits commercialisés notamment cosmétiques, *(*Kirk Othmer Encyclopedia, Cosmetics, Martin M. Rieger, 04/12/2000 ; https://doi.org/10.1002/0471238961.0315191318090507.a01). Au fil des années, des problèmes de conservation ont également entraîné l'introduction d'agent de conservation à spectre vs. les microorganismes contaminants résistants.

En outre, il apparait que certains des agents de conservation couramment utilisés sont inactivés par une variété de tensioactifs. Plus le conservateur est hydrophobe, plus grand est le danger qu'il soit piégé dans des systèmes moléculaires organisés tels que des micelles et subséquemment devenir moins efficaces voire inefficaces contre les microorganismes notamment pathogènes *(*Ullmann's Encyclopedia of Industrial Chemistry, "Skin Cosmetics", G. Schneider et al., vol. 33, p. 221, 2012 Wiley-VCH Verlag GmbH & Co. KGaA, Weinheim DOI: 10.1002/14356007.a24_219).

Ainsi il d'un grand intérêt de proposer de nouvelles associations antimicrobiennes qui relèvent ces défis.

La 4-(3-éthoxy-4-hydroxyphényl)butan-2-one i) est un ingrédient connu comme conservateur de compositions cosmétiques pour protéger les compositions de la contamination microbienne (voir par ex. WO 2011/039445).

Toutefois il est souhaitable de pouvoir incorporer ledit ingrédient i) en concentration réduite dans des compositions, notamment cosmétiques ou dermatologiques, tout en conservant une bonne performance de conservation antimicrobienne. De plus les composés antimicrobiens ne présentent pas toujours une bonne stabilité en composition, et/ou une bonne activité antimicrobienne dans le temps, d'autant plus lorsque le composé antimicrobien est associé à d'autres antimicrobiens qui peuvent avoir des incompatibilités de solubilité, des problèmes d'odeur, de stabilité de composition dans le temps et/ou d'inefficacité antimicrobienne. Il est d'un grand intérêt de mettre à disposition des compositions notamment cosmétiques ou dermatologiques, qui ait non seulement un effet antimicrobien identique voire accru et qui en outre se trouve dans des compositions stables dans les temps, en évitant une modification de l'odeur, tout en conservant dans le temps sa capacité d'agent de conservation i.e. d'efficacité antimicrobienne. En outre il est également intéressant d'avoir un composition qui comprenne plusieurs antimicrobiens qui reste stable en termes de formulation, qui ne soit pas trop visqueuse i.e. inférieure à 45 poises et qui ne change notamment pas d'aspect et/ou de viscosité dans le temps même après plusieurs semaines voire plusieurs mois de stockage à température supérieure ou égale à 25 °C, notamment comprise entre 37 et 45 °C.

Il a été découvert, de façon inattendue, que l'association de : i) la 4-(3-éthoxy-4-hydroxyphényl)butan-2-one ou un de ses sels de bases organiques ou minérales, ainsi que ses solvates tels que les hydrates ; avec ii) la 4-hydroxyacétophénone ou un de ses sels de bases organiques ou minérales, ainsi que ses solvates tels que les hydrates ; permet d'obtenir un mélange antimicrobien présentant une nette amélioration voire une synergie d'activité antimicrobienne. De plus il apparait que la composition comprenant i) et ii) restent stables même après plusieurs semaines voire mois à température ambiante voire même à températures supérieures à 25 °C notamment comprise entre 37 et 45 °C. Par ailleurs il apparait que l'association des deux composés i) et ii) ne présentent pas d'odeur ou une odeur atténuée. Par ailleurs il apparait que ni l'association des ingrédients i) et ii) ni la composition ne présente un changement d'odeur ou une mauvaise odeur qui apparait dans le temps.

L'invention a également pour objet un procédé de traitement cosmétique non thérapeutique des matières kératiniques comprenant l'application sur les matières kératiniques d'une composition, notamment cosmétique, telle que décrite précédemment. Le procédé peut être un procédé cosmétique de soin, de maquillage, de parfumage ou de nettoyage des matières kératiniques.

L'invention a également pour objet un procédé de conservation d'une composition, notamment comprenant un milieu physiologiquement acceptable, en particulier une composition cosmétique ou pharmaceutique, ou d'une composition alimentaire, caractérisé en ce qu'il consiste à incorporer à ladite composition un mélange antimicrobien tel que décrit précédemment.

Les résultats des exemples décrits ci-après montrent l'activité antimicrobienne de l'association de i) + ii) est améliorée d'après les mesures de concentrations minimales inhibitrices (CMI) faites avec plusieurs mélanges, vs. i) seul ou ii) seul à quantité équivalente. L'activité antimicrobienne est considérée comme étant synergique lorsque le mélange antimicrobien permet d'obtenir un pourcentage de croissance de la souche inférieur ou égal à 25 %, voire inférieur ou égal à 20 %.

L'association de la i) 4-(3-éthoxy-4-hydroxyphényl)butan-2-one ainsi que ses sels d'acide ou de base, organique ou minéral, ou ses solvates tels que les hydrates avec la ii) 4-hydroxy-acétophénone ainsi que ses sels d'acide ou de base ou ses solvates tels que les hydrates permet d'obtenir un mélange antimicrobien présentant excellente activité antimicrobienne, en particulier vis-à-vis de *Aspergillus niger, Escherichia coli, Staphylococcus aureus, et Candida albicans*. **Il** apparait en outre que l'amélioration a été obtenue que ce soit avec un support de formule « simplex » ou plus « complexe ». De plus les formules restent stables dans le temps tout en gardant dans le temps une activité antimicrobienne même après 7 jours, 15 jours, 1 mois, et à une température ambiante.

De façon plus précise, l'invention a pour objet un mélange antimicrobien comprenant, ou constitué (ou consistant en), de la i) 4-(3-éthoxy-4-hydroxyphényl)butan-2-one ainsi que ses sels d'acide ou de base, organique ou minéral, et ses solvates tels que les hydrates et la ii) 4-hydroxyacétophénone, ainsi que ses sels d'acide ou de base, organique ou minéral, et les solvates tels que les hydrates l'association de 80 à 90 % en poids, par rapport au poids total de ladite association.

Au sens de la présente invention, et à moins qu'une indication différente ne soit donnée :

- Par « *polymère* épaississant » on entend un polymère qui introduit à 1% en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1 $s^{-1}$. Cette viscosité peut être mesurée à l'aide d'un viscosimètre cône/plan (Rhéomètre Haake R600 ou analogue). Les polymères épaississants peuvent être épaississants de la phase aqueuse et/ou de la phase grasse, préférentiellement de la phase aqueuse ;

- Par polymère épaississant « *organique* » on entend un polymère épaississant tel que défini précédemment qui est constitué de carbone, d'hydrogène, et éventuellement d'azote, d'oxygène, de soufre, d'halogènes tel que le fluor, le chlore, le brome ainsi que du phosphore, des métaux alcalins tels que le sodium, le potassium, ou métaux alcalino-terreux tels que le magnésium ou le calcium. Les polymères organiques selon l'invention ne comprennent pas de silicium ;

- Par l'expression « *polymère épaississant organique non cellulosique »,* on entend selon l'invention, un polymère épaississant organique ne comportant pas de motif cellulose ;

- Par « *tensioactif »* on entend un *« agent de surface* » ou *« surfactant »* qui est un composé susceptible de modifier la tension superficielle entre deux surfaces, les tensioactifs sont des molécules amphiphiles, i.e. qui présentent deux parties de polarité différente, l'une lipophile et apolaire et l'autre hydrophile et polaire ;

- Par « *corps gras »,* on entend, au sens de la présente invention, un composé organique insoluble dans l'eau à température ordinaire (25 °C) et à pression atmosphérique (760 mm de Hg) (solubilité inférieure à 5 %, et de préférence à 1%, encore plus préférentiellement à 0,1 %) ; en outre, les corps gras sont solubles dans les solvants organiques dans les mêmes conditions de température et de pression, comme par exemple dans les solvants halogénés tel que le chloroforme, le dichlorométhane, les alcools inférieurs tels que l'éthanol ou les solvants aromatiques tels que le benzène ou le toluène.

- Par « *sel d'acide organique ou minéral »* on entend plus particulièrement les sels choisis parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : Ar-$S(O)_2OH$ tel que d'acide benzène sulfonique et d'acide toluène sulfonique ; vi) d'acide citrique ; vii) d'acide succinique ; viii) d'acide

tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3C(O)OH$ ; xiv) d'acide triflique $CF_3SO_3H$ et xv) d'acide tétrafluoroborique $HBF_4$ ;

- Par « *sels de bases organiques ou minérale* » on entend les sels de bases ou d'agents alcalins tels que définis ci-après comme les hydroxydes de métal alcalin comme la soude, la potasse, l'ammoniaque, les amines ou les alkanolamines.

- Par *« contre-ion cationique* » on entend un cation ou un groupement cationique issu de sel de base organique ou minérale contrebalançant la charge anionique des ingrédients de formule (I') ou (II') ; plus particulièrement le contre-ion cationique est choisi parmi i) les métaux alcalins tels que sodium, potassium, de préférence $Na^+$, ii) les métaux alcalino-terreux tels que calcium ; iii) ammonium $R_4N^+$ avec R, identique ou différent, représente un atome d'hydrogène, ou un groupe $(C_1\text{-}C_6)$alkyle éventuellement substitué par un ou plusieurs groupes hydroxy, de préférence R représente un atome d'hydrogène ou un groupe $(C_1\text{-}C_4)$alkyle tel que méthyle.

- une chaîne hydrocarbonée est insaturée lorsqu'elle comporte une ou plusieurs liaisons doubles et/ou une ou plusieurs liaisons triples ;

un « radical alkyle » est un radical hydrocarboné saturé en $C_1\text{-}C_{20}$, linéaire ou ramifié, de préférence en $C_1\text{-}C_6$, plus préférentiellement $C_1\text{-}C_4$ tel que méthyle, ou éthyle ;

- un *« radical alkylène »* est un radical divalent hydrocarboné saturé tel que défini précédemment pouvant contenir de 1 à 4 doubles liaisons -C=C-, conjuguées ou non ; particulièrement le groupe alkénylène contient 1 ou 2 insaturation(s) ; l'expression « *éventuellement substitué* » attribué au radical alkyle sous-entend que ledit radical alkyle peut être substitué par un ou plusieurs radicaux choisis parmi les radicaux i) hydroxyle, ii) alkoxy en $C_1\text{-}C_4$, iii) acylamino, iv) amino éventuellement substitué par un ou deux radicaux alkyle, identiques ou différents, en $C_1\text{-}C_4$,;;
- un *« radical alkoxy »* est un radical alkyle-oxy pour lequel le radical alkyle est un radical hydrocarboné, linéaire ou ramifié, en $C_1\text{-}C_{16}$ préférentiellement en $C_1\text{-}C_8$ ;-L'expression « *au moins un* » est équivalente à « *un ou plusieurs »* ; et
- L'expression *« inclusivement* » pour une gamme de concentrations signifie que les bornes de la gamme font partie de l'intervalle défini.

*i) La* 4-(3-éthoxy-4-hydroxyphényl)butan-2-one est un composé de formule (I) :

[Chem. 1]

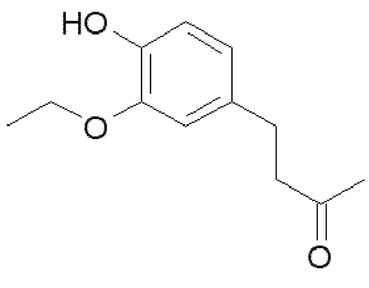

(I).

La i) 4-(3-éthoxy-4-hydroxyphényl)butan-2-one peut se trouver sous forme solvatée, notamment hydratée.
La i) 4-(3-éthoxy-4-hydroxyphényl)butan-2-one peut se trouver sous forme salifiée par une base organique ou minérale de formule (I') suivante :

[Chem. 2]

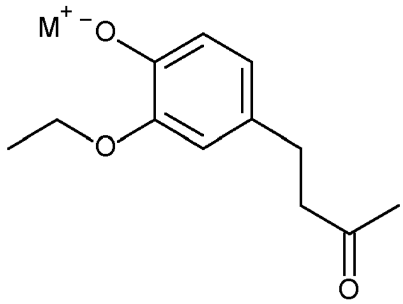

(I')

Formule (I') dans laquelle $M^+$ représente un contre-ion cationique, en particulier un métal alcalin tel que sodium, ou potassium, un métal alcalino-terreux tel que calcium ou un ammonium.

***ii) La 4-hydroxyacétophénone,*** également dénommée 4-hydroxyphényléthanone, p-acétophenol, p-hydroxyphénylméthylcétone, picéol est un composé de formule (II) :

[Chem. 3]

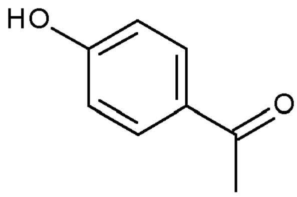

(II).

La ii) 4-hydroxyacétophénone peut se trouver sous forme solvatée, notamment hydratée.

La ii) 4-hydroxyacétophénone peut se trouver sous forme salifiée par une base organique ou minérale de formule (II') suivante :

[Chem. 4]

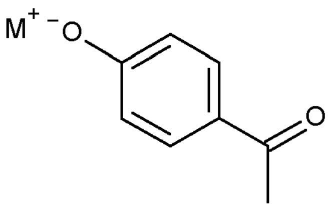

(II')

Formule (II') avec $M^+$ représente un contre-ion cationique, en particulier un métal alcalin tel que sodium, ou potassium, un métal alcalino-terreux tel que calcium ou un ammonium.

Selon un mode de réalisation particulier de l'invention la i) 4-(3-éthoxy-4-hydroxyphényl)butan-2-one et la ii) 4-hydroxyacétophénone sont présents dans ledit mélange ou dans la composition en une teneur telle que le ratio pondéral i) / ii) va de 0,05 à 5, de préférence va de 0,08 à 5, préférentiellement va de 0,08 à 0,25, et de 2 à 4 plus préférentiellement va de 0,15 à 0,25 et de 3 à 3,8. Un tel mélange présente une bonne activité antimicrobienne sur les moisissures, notamment sur *Aspergillus niger.*

Avantageusement, la i) 4-(3-éthoxy-4-hydroxyphényl)butan-2-one et la ii) 4-hydroxyacétophénone sont présents dans ledit mélange ou dans la composition en une teneur telle que le ratio pondéral i) / ii) va de 0,08 à 0,5, de préférence va de 0,08 à 0,3, préférentiellement va de 0,08 à 0,25, plus préférentiellement va de 0,15 à 0,25. Un tel mélange présente une bonne activité antimicrobienne sur les moisissures, notamment sur *Aspergillus niger.*

Selon un autre mode de réalisation particulier de l'invention la i) 4-(3-éthoxy-4-hydroxyphényl)butan-2-one et la ii) 4-hydroxyacétophénone sont présents dans ledit mélange ou dans la composition en une teneur telle que le ratio pondéral i) / ii) de 0,5 à 5, préférentiellement va de 1 à 4 et plus préférentiellement 3 à 3,8. Un tel mélange présente une bonne activité antimicrobienne sur *Aspergillus niger. Escherichia coli, Staphylococcus aureus, et Candida albicans* et ce dans le temps même après plusieurs semaines (1 semaine, 2 semaines voire un mois).

***La composition***

Un autre objet de l'invention est une composition comprenant :

I) une ou plusieurs 4-(3-éthoxy-4-hydroxyphényl)butan-2-one ainsi que ses sels d'acide ou de base, organique ou minéral, et ses solvates tels que les hydrates et
ii) une ou plusieurs 4-hydroxyacétophénone, ainsi que ses sels d'acide ou de base, organique ou minéral, et les solvates tels que les hydrates.

La composition est un milieu physiologiquement acceptable. La composition est notamment une composition cosmétique ou pharmaceutique ou dermatologique. La composition peut également être une composition alimentaire (nourriture).

*Le milieu physiologiquement acceptable :*

On entend par « milieu physiologiquement acceptable » un milieu approprié pour être appliqué sur les matières kératiniques, appelé aussi support de formule, qui est un milieu cosmétique ou pharmaceutique généralement constitué par de l'eau ou par un mélange d'eau et d'un ou plusieurs solvants organiques ou par un mélange de solvants organiques. De préférence la composition comprend de l'eau et dans une teneur comprise inclusivement notamment entre 5% et 99,9 % par rapport au poids total de la composition, plus préférentiellement entre 10 et 90 %, encore plus préférentiellement entre 20 % et 80 % en poids par rapport au poids total de la composition.

*Les Solvants organiques:*

Par *« solvant organique »,* on entend une substance organique capable de dissoudre une autre substance sans la modifier chimiquement.
A titre de solvant organique, on peut par exemple citer a) les alcanols en $C_2$-$C_6$, tels que l'éthanol et l'isopropanol ; b) les polyols miscibles à l'eau à la température ambiante (25 °C) notamment choisi parmi les polyols ayant notamment de 2 à 10 atomes de carbones, de préférence ayant de 2 à 6 atomes de carbone, tels que la glycérine, le propylène glycol, le 1,3-propanediol, le butylène glycol, le pentylène glycol, l'hexylène glycol, le dipropylène glycol, le diéthylène glycol, la diglycérine ; c) les éthers de polyols comme le 2-butoxyéthanol, le monométhyléther de propylèneglycol, le monoéthyléther et le monométhyléther du diéthylèneglycol, ainsi que d) les alcools aromatiques comme l'alcool benzylique ou le phénoxyéthanol, et leurs mélanges.
Selon un mode de réalisation particulier la composition comprend en outre un ou plusieurs polyols notamment choisi parmi les polyols ayant notamment de 2 à 10 atomes de carbones, de préférence ayant de 2 à 6 atomes de carbone, tels que la glycérine.
Dans un mode de réalisation préféré, la 4-(3-éthoxy-4-hydroxyphényl)butan-2-one est utilisée en combinaison avec une quantité efficace d'au moins un solvant organique qui peut être choisi parmi l'éthanol, le 1,2-propylèneglycol, le 1,3-propanediol, le PEG-8 (polyéthylène glycol contenant 8 motifs d'éthylène glycol), le propylène carbonate, le dipropylène glycol, le 1,2-hexylène glycol, le PEG-4 .
De préférence, le solvant organique est choisi parmi l'éthanol, le 1,2-propylèneglycol, le 1,3-propanediol, le PEG-8, le propylène carbonate.
Avantageusement, la composition selon l'invention comprend du 1,3-propanediol, notamment en une teneur allant de 0,1 à 20 % en poids, par rapport au poids total de la composition, et de préférence allant de 0,1 à 10 % en poids, préférentiellement allant de 0,5 à 5 % en poids.
Pour la composition alimentaire on privilégiera l'eau et comme solvants organiques ceux propres à la consommation tels que l'éthanol.
Les solvants organiques sont, de préférence, présents dans des proportions de préférence comprise inclusivement entre 0,1 et 40 % en poids environ par rapport au poids total de la composition, et plus préférentiellement entre 1 et 20 % en poids environ et encore plus particulièrement compris inclusivement entre 5% et 10 % en poids par rapport au poids total de la composition.

*Le pH :*

Le pH de la composition selon l'invention est généralement compris inclusivement entre 2 et 12 environ, et de préférence entre 3 et 11 environ. Il peut être ajusté à la valeur désirée au moyen d'agents acidifiants ou alcalins habituellement utilisés en teinture des fibres kératiniques ou bien encore à l'aide de systèmes tampons classiques.
Le pH de la composition est préférentiellement compris inclusivement entre 6 et 9, particulièrement entre 7 à 9 et plus particulièrement autour du pH neutre 7.
Parmi les agents acidifiants, on peut citer, à titre d'exemple, les acides minéraux ou organiques tels que définis précédemment notamment l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les bases ou agents alcalins on peut citer, à titre d'exemple, l'ammoniaque, les carbonates alcalins, les alcanolamines et autres agents alcalins tels que définis ci-après, de préférence les alcanolamines telles que les mono-, di- et triéthanolamines.

*Le ou les tensioactifs*

Selon un mode de réalisation particulier de l'invention la composition comprend un ou plusieurs tensioactifs. Le ou les tensioactifs peuvent être non ioniques, anioniques, cationiques, zwitterioniques ou amphotères, de préférence le ou les tensioactifs sont non ioniques ou anioniques.
Parmi les tensioactifs non ioniques selon l'invention on peut citer, seuls ou mélanges, a) les alcools gras, b) les alpha-diols, c) les alkylphénols ces 3 types composés a) à c) étant polyéthoxylés, polypropoxylés et/ou polyglycérolés, et ayant une chaîne grasse comportant par exemple 8 à 30 atomes de carbone, en particulier comportant 10 à 22 atome de carbone, le nombre de groupements oxyde d'éthylène ou oxyde de propylène pouvant aller notamment de 2 à 200, en particulier de 10 à 100 et le nombre de groupements glycérol pouvant aller notamment de 2 à 200, en particulier de 10 à 100. On peut également citer les copolymères d'oxyde d'éthylène (OE) et de propylène (OP), les condensats d'oxyde d'éthylène et de propylène sur des alcools gras ; les amides gras polyéthoxylés ayant de préférence de 2 à 30 moles OE, les amides gras polyglycérolés comportant en moyenne 1 à 5 groupements glycérol et en particulier 1,5 à 4 ; les esters d'acides gras du sorbitan oxyéthylénés ayant de 2 à 200 moles OE, en particulier de 10 à 100 OE; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine.
De préférence, le tensioactif non ionique est choisi parmi : les alcools gras (poly)éthoxylés ; les alcools gras glycérolés ; les alkylpolyglycosides. de préférence les mono et diesters d'acides gras du sorbitan oxyéthylénés ayant de 2 à 200 moles OE, en particulier de 10 à 100 OE.
Pus préférentiellement les tensioactifs sont choisis parmi les mono et diesters d'acides gras du sorbitan oxyéthylénés ayant de 2 à 200 moles OE, en particulier de 10 à 100 OE tel que le stéarate de propylène glycol ayant de 10 à 30 OE tel que 20 OE; les mono distéarate de glycéryle / stéarate de polyéthylène glycol (100 OE).
Par chaîne grasse, on entend une chaîne hydrocarbonée comprenant 6 à 30 atomes de carbone, de préférence de 8 à 24 atomes de carbone, linéaire ou ramifiée, saturée ou non, telle que stéaryle.
En ce qui concerne les alkypolyglycosides, ces composés sont bien connus et peuvent être plus particulièrement représenté par la formule générale suivante : $R_1O-(R_2O)_t\ (G)_v$ (III) Formule (III) dans laquelle :

- $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone ;
- $R_2$ représente un radical alkylène comportant environ de 2 à 4 atomes de carbone ;
- G représente un motif sucre comportant de 5 à 6 atomes de carbone ;
- t désigne un entier compris inclusivement entre 0 et 10, de préférence entre 0 et 4, de préférence entre 0 et 4 ; et
- v désigne un entier compris inclusivement 1 et 15.

Des alkylpolyglycosides préférés selon la présente invention sont des composés de formule (III) dans laquelle $R_1$ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (III), peut aller de 1 à 15, de préférence de 1 à 4. Le degré moyen de polymérisation est plus particulièrement compris entre 1 et 2 et encore plus préférentiellement de 1,1 à 1,5.
Les liaisons glycosidiques entre les motifs sucre sont de type 1-6 ou 1-4 et de préférence 1-4.
Des composés de formule (III) sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et

TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.

On peut également utiliser par exemple, l'Alkyl en $C_8/C_{16}$ polyglucoside 1,4 en solution aqueuse à 53% commercialisé par COGNIS sous la référence PLANTACARE® 818 UP.

En ce qui concerne les tensioactifs mono ou polyglycérolés, ils comportent de préférence en moyenne de 1 à 40 groupements glycérol, plus particulièrement de 10 à 30 groupements glycérol tel que 20.

Selon un mode de réalisation particulier de l'invention les tensioactifs sont monoglycérolés ou polyglycérolés sont de préférence choisis parmi les composés de formules suivantes :

$$RO[CH_2CH(CH_2OH)O]_mH,$$

$$RO[CH_2CH(OH)CH_2O]_mH \text{ ou}$$

$$RO[CH(CH_2OH)CH_2O]_mH \text{ ;}$$

Formules dans lesquelles :

- R représente un radical hydrocarboné saturé ou insaturé, linéaire ou ramifié, comportant de 8 à 40 atomes de carbone et de préférence de 10 à 30 atomes de carbone ;
- m est un nombre compris entre 1 et 30, de préférence entre 1 et 10, plus particulièrement de 1,5 à 6. R peut éventuellement comprendre des hétéroatomes tels que par exemple oxygène et azote. En particulier, R peut éventuellement comprendre un ou plusieurs groupements hydroxy et/ou éther et/ou amide. R désigne de préférence des radicaux alkyle et/ou alkényle en $C_{10}$-$C_{20}$, éventuellement mono ou polyhydroxylé.

De préférence la composition de l'invention comprend un ou plusieurs alcools gras (poly)éthoxylés convenant à la mise en œuvre de l'invention sont plus particulièrement choisis parmi les alcools comportant de 8 à 30 atomes de carbone, et de préférence de 12 à 22 atomes de carbone.

Les alcools gras (poly)éthoxylés présentent plus particulièrement un ou plusieurs groupes hydrocarbonés, linéaires ou ramifiés, saturés ou insaturés, comprenant 8 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

Le ou les alcools gras (poly)éthoxylés sont de préférence de formule suivante :

$$R^a\text{-}[O\text{-}CH_2\text{-}CH_2]_n\text{-}OH$$

avec

- $R^a$ représentant un groupe alkyle linéaire ou ramifié en $C_1$-$C_{40}$, ou alkényle linéaire ou ramifié en $C_2$-$C_{30}$ (préférentiellement alkyle en $C_8$-$C_{30}$) ; et
- n représente un entier compris inclusivement entre 1 et 200, préférentiellement entre 2 et 100, plus particulièrement compris inclusivement entre 10 et 50, encore plus particulièrement compris inclusivement entre 15 et 30 tel que 100 ou 20.

Les alcools gras (poly)éthoxylés sont plus particulièrement les alcools gras comportant de 8 à 22 atomes de carbone et oxyéthylénés par 1 à 30 moles d'oxyde d'éthylène (1 à 100 OE). Parmi eux, on peut citer plus particulièrement l'alcool laurique 20 OE, l'alcool laurique 30 OE, l'alcool décylique 3 OE, l'alcool décylique 5 OE et l'alcool oléique 20 OE.

On peut utiliser aussi des mélanges de ces alcools gras (poly)oxyéthylénés.

Parmi les tensioactifs non-ioniques on utilise de préférence les alkyl $C_6$-$C_{24}$ polyglucosides et les alcools gras (poly)éthoxylés et on utilise plus particulièrement les alkyl $C_8$-$C_{16}$ polyglucoside.

La quantité de tensioactifs non ioniques va de préférence de 0,5 % à 20 % en poids, en particulier de 1 % à 10 % en poids, et plus particulièrement de 2 à 5 % en poids rapportée au poids total de la composition de l'invention.

Selon un autre mode de réalisation particulier de l'invention, la composition comprend un ou plusieurs agents tensioactifs anioniques.

On entend par « *agent tensioactif anionique »,* un tensioactif ne comportant à titre de groupements ioniques ou ionisables que des groupements anioniques. Ces groupements anioniques sont choisis de préférence parmi les groupements $-C(O)OH$, $-C(O)O^-$, $-SO_3H$, $-S(O)_2O^-$, $-OS(O)_2OH$, $-OS(O)_2O^-$, $-P(O)_2OH$, $-P(O)_2O^-$, $-P(O)O_2^-$, $-P(OH)_2$,

=P(O)OH, -P(OH)O$^-$, =P(O)O$^-$, =POH, =PO$^-$, les parties anioniques comprenant un contre ion cationique tel que un métal alcalin, un métal alcalino-terreux, ou un ammonium, plus préférentiellement les groupements sont carboxy-C(O)OH, ou carboxylate -C(O)O$^-$.

A titre d'exemples d'agents tensioactifs anioniques utilisables dans la composition selon l'invention, on peut citer acides alkylcarboxyliques, les alkyl sulfates, les alkyl éther sulfates, les alkylamidoéthersulfates, les alkylarylpolyéthersulfates, les monoglycéride-sulfates, les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les sels de diesters d'alkyle et d'acides polyglycoside-polycarboxyliques, les acyllactylates, les sels d'acides D-galactoside-uroniques, les sels d'acides alkyl éther-carboxyliques, les sels d'acides alkylaryl éther-carboxyliques, les sels d'acides alkyl amidoéther-carboxyliques, et les formes non salifiées correspondantes de tous ces composés, les groupes alkyle et acyle de tous ces composés comportant de 8 à 30 atomes de carbone, de préférence de 10 à 22 atomes de carbone et le groupe aryle désignant un groupe phényle.

Ces composés peuvent être oxyéthylénés et comportent alors de préférence de 1 à 50 motifs oxyde d'éthylène.

Les sels de monoesters d'alkyle en $C_6$-$C_{24}$ et d'acides polyglycoside-polycarboxyliques peuvent être choisis parmi les polyglycoside-citrates d'alkyle en $C_6$-$C_{24}$, les polyglycosides-tartrates d'alkyle en $C_6$-$C_{24}$ et les polyglycoside-sulfosuccinates d'alkyle en $C_6$-$C_{24}$.

Lorsque l'agent ou les agents tensioactifs anioniques sont sous forme de sel, il(s) peu(ven)t être choisi(s) parmi les sels de métaux alcalins tels que le sel de sodium ou de potassium et de préférence de sodium, les sels d'ammonium, les sels d'amines et en particulier d'aminoalcools ou les sels de métaux alcalino-terreux tel que les sels de magnésium.

A titre d'exemple de sels d'aminoalcools, on peut citer notamment les sels de mono-, di- et triéthanolamine, les sels de mono-, di- ou triisopropanolamine, les sels de 2-amino-2-méthyl-1-propanol, 2-amino-2-méthyl-1,3-propanediol et tris(hydroxy-méthyl)amino méthane.

On utilise de préférence les sels de métaux alcalins ou alcalinoterreux et en particulier les sels de sodium ou de magnésium.

Parmi les agents tensioactifs anioniques cités, on préfère utiliser les acides alkyl($C_6$-$C_{24}$)carboxyliques, en particulier acides alkyl($C_{10}$-$C_{20}$)carboxyliques, de préférence d'origine naturelle en particulier végétale tel que acide stéarique, pouvant être sous forme de sels de métaux alcalins, d'ammonium, d'aminoalcools, et de métaux alcalino-terreux, ou un mélange de ces composés.

La quantité de tensioactifs anioniques va de préférence de 0,5 % à 20 % en poids, en particulier de 1 % à 10 % en poids, et plus particulièrement de 2 à 5 % en poids rapportée au poids total de la composition de l'invention.

La quantité de tensioactifs va de préférence de 0,5 % à 30 % en poids, en particulier de 1 % à 20 % en poids, et plus particulièrement de 2 à 10 % en poids, plus préférentiellement entre 4 et 6 % rapportée au poids total de la composition de l'invention.

*Le ou les corps gras*

La composition de l'invention comprend un ou plusieurs corps gras. Les corps gras de l'invention ne sont pas oxyalkylénés.

De préférence, les corps gras de l'invention sont choisis parmi les hydrocarbures, les alcools gras, les esters gras, les silicones et les éthers gras ou leurs mélanges.

Les corps gras de l'invention peuvent être liquides ou non liquides à la température ambiante (25 °C) et à pression atmosphérique (760 mm de Hg ; soit 1,013.105 Pa).

Les corps gras liquides de l'invention présentent de préférence une viscosité inférieure ou égale à 2 Pa.s mieux inférieure ou égale à 1 Pa.s et encore mieux inférieure ou égale à 0,1 Pa.s à la température de 25 °C et à un taux de cisaillement de 1 s-1.

Par hydrocarbure liquide, on entend, un hydrocarbure composé uniquement d'atomes de carbone et d'hydrogène liquide à la température ordinaire (25 °C) et à pression atmosphérique (760 mm de Hg ; soit 1,013.105 Pa).

Plus particulièrement, les hydrocarbures liquides sont choisis parmi :

- les alcanes en $C_6$-$C_{16}$ linéaires ou ramifiés, éventuellement cycliques. A titre d'exemple, on peut citer l'hexane, l'undécane, le dodécane, le tridécane, les isoparaffines comme l'isohexadécane, l'isododécane et l'isodécane.
- les hydrocarbures linéaires ou ramifiés, d'origine minérale animale ou synthétique de plus de 16 atomes de carbone, tels que les huiles de paraffine, l'huile de vaseline, les polydécènes, le polyisobutène hydrogéné tel que Parléam®, le squalane.

Dans une variante préférée, le ou les hydrocarbures liquides sont choisis parmi les huiles de paraffine, l'huile de

vaseline.

Par alcool gras liquide, on entend, un alcool gras non glycérolé et non oxyalkyléné et liquide à la température ordinaire (25 °C) et à pression atmosphérique (760 mm de Hg ; soit 1,013.105 Pa).

De préférence, les alcools gras liquides de l'invention comportent de 8 à 30 atomes de carbone, plus préférentiellement en $C_{10}$-$C_{22}$, encore plus préférentiellement en $C_{14}$-$C_{20}$, mieux en $C_{16}$-$C_{18}$.

Les alcools gras liquides de l'invention peuvent être saturés ou insaturés.

Les alcools gras liquides saturés sont de préférence ramifiés. Ils peuvent éventuellement comprendre dans leurs structure au moins un cycle aromatique ou non. De préférence, ils sont acycliques.

Plus particulièrement, les alcools gras saturés liquides de l'invention sont choisis parmi l'octyldodécanol, l'alcool isostéarylique, le 2-hexyldécanol.

Selon une autre variante de l'invention le ou les corps gras sont choisi parmi les alcools gras insaturés liquides. Ces alcools gras insaturés liquides présentent dans leur structure au moins une double ou triple liaison. De préférence, les alcools gras de l'invention possèdent dans leur structure une ou plusieurs doubles liaisons. Lorsque plusieurs double liaisons sont présentes, elles sont de préférence au nombre de 2 ou 3 et elles peuvent être ou non conjuguées.

Ces alcools gras insaturés peuvent être linéaires ou ramifiés.

Ils peuvent éventuellement comprendre dans leurs structure au moins un cycle aromatique ou non. De préférence, ils sont acycliques.

Plus particulièrement, les alcools gras insaturés liquides de l'invention sont choisis parmi l'alcool oléique (ou oléylique), l'alcool linoléique (ou linoléylique), l'alcool linolénique (ou linolénylique), l'alcool undécylénique.

L'alcool oléique est tout particulièrement préféré.

Par esters gras liquide, on entend, un ester issu d'un acide gras et / ou d'un alcool gras et liquide à la température ordinaire (25 °C) et à pression atmosphérique (760 mm de Hg ; soit 1,013.105 Pa).

Les esters sont de préférence les esters liquides de mono ou polyacides aliphatiques saturés ou insaturés, linéaires ou ramifiés en $C_1$-$C_{26}$ et de mono ou polyalcools aliphatiques saturés ou insaturés, linéaires ou ramifiés en $C_1$-$C_{26}$, le nombre total d'atomes de carbone des esters étant supérieur ou égal à 10.

De préférence, pour les esters de monoalcools, l'un au moins de l'alcool ou de l'acide dont sont issus les esters de l'invention est ramifié.

Parmi les monoesters de monoacides et de monoalcools, on peut citer les palmitates d'éthyle et d'isopropyle, les myristates d'alkyles tels que le myristate d'isopropyle, d'éthyle, le stéarate d'isocétyle, l'isononanoate d'éthyl-2-hexyle, le néopentanoate d'isodécyle, le néopentanoate d'isostéaryle, et les (iso)stéarates d'alkyles en $C_{10}$-$C_{22}$, de préférence en $C_{12}$-$C_{20}$ tels que l'isostéarate d'isopropyle.

On peut également utiliser les esters d'acides di ou tricarboxyliques en $C_4$-$C_{22}$ et d'alcools en $C_1$-$C_{22}$ et les esters d'acides mono di ou tricarboxyliques et d'alcools non sucres di, tri, tétra ou pentahydroxy en $C_4$-$C_{26}$.

On peut notamment citer : le sébacate de diéthyle ; le sébacate de diisopropyle ; le sébacate de di(2éthylhexyle) ; l'adipate de diisopropyle ; l'adipate de di n-propyle ; l'adipate de dioctyle ; l'adipate de di(2éthylhexyle) ; l'adipate de diisostéaryle ; le maléate de di(2éthylhexyle) ; le citrate de triisopropyle ; le citrate de triisocétyle ; le citrate de trisostéaryle ; le trilactate de glycéryle ; le trioctanoate de glycéryle ; le citrate de trioctyldodécyle ; le citrate de trioléyle; le diheptanoate de néopentyl glycol ; le diisononate de diéthylène glycol.

La composition peut également comprendre, à titre d'ester gras liquide, des esters et di-esters de sucres d'acides gras en $C_6$-$C_{30}$, de préférence en $C_{12}$-$C_{22}$. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en $C_6$-$C_{30}$, de préférence en $C_{12}$-$C_{22}$, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléopalmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui

est un dioléate de méthylglucose.

Enfin, on peut aussi utiliser les esters naturels ou synthétiques de mono, di ou triacides avec le glycérol.

Parmi ceux-ci on peut citer les huiles végétales.

Comme huiles d'origine végétale ou triglycérides synthétiques, utilisables dans la composition de l'invention à titre d'esters gras liquides, on peut citer par exemple :

- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique / caprique comme ceux vendus par la société Stearineries Dubois ou ceux vendus sous les dénominations Miglyol® 810, 812 et 818 par la société Dynamit Nobel, l'huile de jojoba, l'huile de beurre de karité.

De préférence, on utilisera à titre d'esters selon l'invention des esters gras liquides issus de monoalcools.

Les myristate ou palmitate d'isopropyle sont préférés.

Par silicone liquide, on entend, un organopolysiloxane liquide à la température ordinaire (25 °C) et à pression atmosphérique (760 mm de Hg ; soit 1,013.105 Pa).

De préférence, la silicone est choisie parmi les polydialkylsiloxanes liquides, notamment les polydiméthylsiloxanes (PDMS) liquides, et les polyorganosiloxanes liquides comportant au moins un groupement aryle.

Ces silicones peuvent être aussi organomodifiées. Les silicones organomodifiées utilisables conformément à l'invention sont des silicones liquides telles que définies précédemment et comportant dans leur structure un ou plusieurs groupements organofonctionnels fixés par l'intermédiaire d'un groupe hydrocarboné.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60 °C et 260 °C, et plus particulièrement encore parmi :

(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, le dodécaméthylcyclopentasiloxane commercialisé sous le nom de SILSOFT 1217 par MOMENTIVE PERFORMANCE MATERIALS, ou le cyclohexadiméthylsiloxane ainsi que leurs mélanges.

On peut également citer les cyclocopolymères du type diméthylsiloxanes / méthylalkylsiloxane, tel que la SILICONE VOLATILE® FZ 3109 commercialisée par la société UNION CARBIDE, de formule :

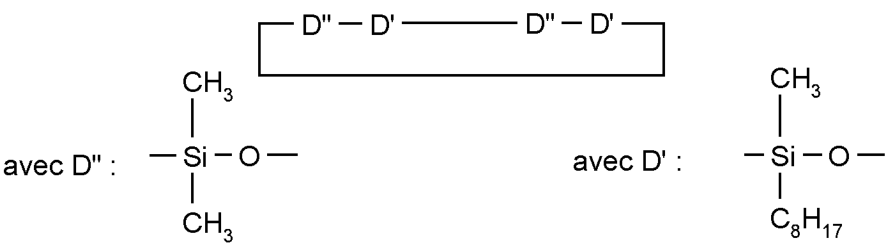

avec D'' :

avec D' :

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane.

(ii) les polydialkylsiloxanes volatiles linéaires ayant 2 à 9 atomes de silicium et présentant une viscosité inférieure ou égale à 5.10-6 $m^2/s$ à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics". La viscosité des silicones est mesurée à 25 °C selon la norme ASTM 445 Appendice C.

On peut utiliser aussi des polydialkylsiloxanes non volatiles.

Ces silicones non volatiles sont plus particulièrement choisies parmi les polydialkylsiloxanes parmi lesquels on peut citer principalement les polydiméthylsiloxanes à groupements terminaux triméthylsilyl.

Parmi ces polydialkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Parmi les silicones à groupements aryle figurent des polydiaryl siloxanes, notamment des polydiphénylsiloxanes, et des polyalkyl-arylsiloxanes. On peut citer à titre d'exemple les produits commercialisés sous les dénominations suivantes :

- les huiles SILBIONE® de la série 70 641 de RHODIA ;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- certaines huiles des séries SF de GENERAL ELECTRIC telles que SF 1023, SF 1154, SF 1250, SF 1265.

Les silicones liquides organomodifiées peuvent notamment posséder des groupements polyéthylèneoxy et / ou polypropylèneoxy. On peut ainsi citer la silicone KF-6017 proposé par SHIN ETSU et les huiles SILWET® L 722, L 77 de la société UNION CARBIDE.

Les éthers gras liquides sont choisis parmi les dialkyléthers liquides tels que le dicaprylyléther.

Les corps gras peuvent être non liquides à température ambiante et à pression atmosphérique.

Par non liquides, on entend de préférence un composé solide ou un composé présentant une viscosité supérieure à 2 Pa.s à la température de 25 °C et à un taux de cisaillement de 1 s-1.

Plus particulièrement, les corps gras non liquides sont choisis parmi les alcools gras les esters d'acide gras et / ou d'alcool gras, les cires non siliconées, les silicones, les éthers gras, non liquides et de préférence solides.

Les alcools gras non liquides convenant à la mise en œuvre de l'invention sont plus particulièrement choisis parmi les alcools saturés ou insaturés, linéaires ou ramifiés, comportant de 8 à 30 atomes de carbone, plus préférentiellement en $C_{10}$-$C_{22}$, encore plus préférentiellement en $C_{14}$-$C_{20}$, mieux en $C_{16}$-$C_{18}$..

L'alcool cétylique et l'alcool stéarylique et leur mélange (alcool cétylstéarylique).sont tout particulièrement préférés.

En ce qui concerne les esters d'acide gras et / ou d'alcools gras non liquides, on peut citer notamment les esters solides issus d'acides gras en $C_9$-$C_{26}$ et d'alcools gras en $C_9$-$C_{26}$.

Parmi ces esters, on peut citer le béhénate d'octyldodécyle ; le béhénate d'isocétyle ; le lactate de cétyle ; l'octanoate de stéaryle ; l'octanoate d'octyle ; l'octanoate de cétyle ; l'oléate de décyle ; le stéarate de myristyle ; le palmitate d'octyle ; le pélargonate d'octyle ; le stéarate d'octyle ; les myristates d'alkyles tels que le myristate de cétyle, de mirystyle, de stéaryle ; le stéarate d'hexyle, plus particulièrement le myrystate de myristyle..

Toujours dans le cadre de cette variante, on peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; le maléate de dioctyle.

Parmi tous les esters additionnels cités ci-dessus, on préfère utiliser les palmitates de myristyle, de cétyle, de stéaryle, les myristates d'alkyles tels que le myristate de cétyle, le myristate de stéaryle myristyle.

La cire ou les cires (non siliconées) sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société **BERTIN** (France), les cires animales comme les cires d'abeilles, les cires d'abeilles modifiées (cerabellina), les cires d'abeilles blanches telles que celles vendues par KOSTER KEUNEN ; d'autres cires ou matières premières cireuses utilisables

selon l'invention sont notamment les cires marines telles que celle vendue par la Société SOPHIM sous la référence M82, les cires de polyéthylène ou de polyoléfines en général.

Les silicones non liquides conformément à l'invention peuvent se présenter sous forme de cires, de résines ou de gommes.

De préférence, la silicone non liquide est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de 5.10 6 $m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

$$R_2SiO_{2/2}, R_3SiO_{1/2}, RSiO_{3/2} \text{ et } SiO_{4/2}$$

Formules dans lesquelles :

R, identique ou différent, de préférence identique, représente un alkyl possédant 1 à 16 atomes de carbone. Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl / triméthyl siloxane.

On peut également citer les résines du type triméthylsiloxysilicate commercialisées notamment sous les dénominations X22-4914, X21-5034 et X21-5037 par la société SHIN-ETSU.

Parmi les silicones organomodifiées additionnelles, on peut citer les polyorganosiloxanes comportant :

- des groupements aminés substitués ou non comme les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;

- des groupements alcoxylés, comme le produit commercialisé sous la dénomination ABIL WAX® 2428, 2434 et 2440 par la société GOLDSCHMIDT.

Les éthers gras non liquides sont choisis parmi les dialkyléthers et notamment le dicétyléther, et le distéaryl éther, seuls ou en mélange.

La composition selon l'invention peut comprendre un ou plusieurs beurres, identiques ou différents, de préférence d'origine végétale.

Selon une mode préféré de l'invention la teneur pondérale du ou des beurres selon l'invention, en acides gras $C_{16}$ des triglycérides exprimée par rapport à la totalité des acides gras des triglycérides est inférieure à 23%.

Par « *beurre* » (également appelé « corps gras pâteux ») au sens de la présente invention, on entend un composé gras lipophile à changement d'état solide/liquide réversible et comportant à la température de 25 °C une fraction liquide et une fraction solide, et à pression atmosphérique (760 mm Hg) En d'autres termes, la température de fusion commençante

du composé pâteux peut être inférieure à 25 °C. La fraction liquide du composé pâteux mesurée à 25 °C peut représenter 9 à 97 % en poids du composé. Cette fraction liquide à 25 °C représente de préférence entre 15 et 85 %, de préférence encore entre 40 et 85 % en poids.

De préférence, le ou les beurres présentent une température de fin de fusion inférieure à 60 °C.

De préférence, le ou les beurres présentent une dureté inférieure ou égale à 6 MPa.

De préférence, les corps gras pâteux présentent à l'état solide une organisation cristalline anisotrope, visible par observations aux rayons X.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination "DSC Q2000" par la société TA Instruments.

Concernant la mesure de la température de fusion et la détermination de la température de fin de fusion, les protocoles de préparation des échantillons et de mesure sont les suivants :

Un échantillon de 5 mg de corps gras pâteux préalablement chauffé à 80°C et prélevés sous agitation magnétique à l'aide d'une spatule également chauffée est placé dans une capsule hermétique en aluminium, ou creuset. Deux essais sont réalisés pour s'assurer de la reproductibilité des résultats.

Les mesures sont réalisées sur le calorimètre mentionné ci-dessus. Le four est soumis à un balayage d'azote. Le refroidissement est assuré par l'échangeur thermique RCS 90. L'échantillon est ensuite soumis au protocole suivant en étant tout d'abord mis en température à 20 °C, puis soumis à une première montée en température allant de 20 °C à 80 °C, à la vitesse de chauffe de 5 °C/minute, puis est refroidi de 80 °C à -80 °C à une vitesse de refroidissement de 5 °C/minute et enfin soumis à une deuxième montée en température allant de -80 °C à 80 °C à une vitesse de chauffe de 5 °C/minute. Pendant la deuxième montée en température, on mesure la variation de la différence de puissance absorbée par le creuset vide et par le creuset contenant l'échantillon de beurre en fonction de la température. Le point de fusion du composé est la valeur de la température correspondant au sommet du pic de la courbe représentant la variation de la différence de puissance absorbée en fonction de la température.

La température de fin de fusion correspond à la température à laquelle 95% de l'échantillon a fondu.

La fraction liquide en poids du beurre à 25 °C est égale au rapport de l'enthalpie de fusion consommée à 25 °C sur l'enthalpie de fusion du beurre.

L'enthalpie de fusion du composé pâteux est l'enthalpie consommée par le composé pour passer de l'état solide à l'état liquide. Le beurre est dit à l'état solide lorsque l'intégralité de sa masse est sous forme solide cristalline. Le beurre est dit à l'état liquide lorsque l'intégralité de sa masse est sous forme liquide.

L'enthalpie de fusion du beurre est égale à l'intégrale de l'ensemble de la courbe de fusion obtenue à l'aide du calorimètre suscité, avec une montée en température de 5 ou 10 °C par minute, selon la norme ISO 11357-3:1999. L'enthalpie de fusion du beurre est la quantité d'énergie nécessaire pour faire passer le composé de l'état solide à l'état liquide. Elle est exprimée en J/g.

L'enthalpie de fusion consommée à 25 °C est la quantité d'énergie absorbée par l'échantillon pour passer de l'état solide à l'état qu'il présente à 25 °C constitué d'une fraction liquide et d'une fraction solide.

La fraction liquide du beurre mesurée à 32 °C représente de préférence de 30 à 100 % en poids du composé, de préférence de 50 à 100 %, de préférence encore de 60 à 100 % en poids du composé. Lorsque la fraction liquide du beurre mesurée à 32 °C est égale à 100 %, la température de la fin de la plage de fusion du composé pâteux est inférieure ou égale à 32 °C.

La fraction liquide du beurre mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Concernant la mesure de la dureté, les protocoles de préparation des échantillons et de mesure sont les suivants :

La composition selon l'invention ou le beurre est placé dans un moule de 75 mm de diamètre qui est rempli à environ 75 % de sa hauteur. Afin de s'affranchir du passé thermique et de contrôler la cristallisation, le moule est placé à l'étuve programmable Vötsch VC0018 où il est tout d'abord mis en température à 80°C pendant 60 minutes, puis refroidi de 80 °C à 0 °C à une vitesse de refroidissement de 5 °C/minute, puis laissé à la température stabilisée de 0 °C pendant 60 minutes, puis soumis à une montée en température allant de 0 °C à 20 °C, à une vitesse de chauffe de 5 °C/minute, puis laissé à la température stabilisée de 20 °C pendant 180 minutes.

La mesure de la force de compression est réalisée avec le texturomètre TA/TX2i de Swantech. Le mobile utilisé est choisi selon la texture :

- mobile cylindrique en acier de 2 mm de diamètre pour les matières premières très rigides ;
- mobile cylindrique en acier de 12 mm de diamètre pour les matières premières peu rigides ;

La mesure comporte 3 étapes :

- une 1ère étape après détection automatique de la surface de l'échantillon où le mobile se déplace à la vitesse de mesure de 0,1 mm/s, et pénètre dans la composition selon l'invention ou le beurre à une profondeur de pénétration de 0,3 mm, le logiciel note la valeur de la force maximale atteinte ;
- une 2ème étape dite de relaxation ou le mobile reste à cette position pendant une seconde et où on note la force après 1 seconde de relaxation ; enfin
- une 3ème étape dite de retrait ou le mobile revient à sa position initiale à la vitesse de 1 mm/s et on note l'énergie de retrait de la sonde (force négative).

La valeur de la dureté mesurée lors de la première étape correspond à la force de compression maximale mesurée en Newton divisée par la surface du cylindre du texturomètre exprimée en $mm^2$ en contact avec le beurre ou la composition selon l'invention. La valeur de dureté obtenue est exprimée en méga-pascals ou MPa.

Selon un mode préféré de l'invention le ou les beurres particuliers sont d'origine végétale tels que ceux décrit dans Ullmann's Encyclopedia of Industrial Chemistry (« Fats and Fatty Oils», A. Thomas, Published Online : 15 JUN 2000, DOI: 10.1002/14356007.a10_173, point 13.2.2.2. Shea Butter, Borneo Tallow, and Related Fats (Vegetable Butters)).

On peut citer plus particulièrement le beurre de karité, le beurre de Karité Nilotica (Butyrospermum parkii), le beurre de Galam, (Butyrospermum parkii), le beurre ou graisse de Bornéo ou tengkawang tallow) (Shorea stenoptera), beurre de Shorea, beurre d'Illipé , beurre de Madhuca ou Bassia Madhuca longifolia, beurre de mowrah (Madhuca Latifolia), beurre de Katiau (Madhuca mottleyana), le beurre de Phulwara (M. butyracea), le beurre de mangue (Mangifera indica), le beurre de Murumuru (Astrocaryum murumuru), le beurre de Kokum (Garcinia Indica), le beurre d'Ucuuba (Virola sebifera), le beurre de Tucuma, le beurre de Painya (Kpangnan) (Pentadesma butyracea), le beurre de café (Coffea arabica), le beurre d'abricot (Prunus Armeniaca), le beurre de Macadamia (Macadamia Ternifolia), le beurre de pépin de raisin (Vitis vinifera), le beurre d'avocat (Persea gratissima), le beurre d'olives (Olea europaea), le beurre d'amande douce (Prunus amygdalus dulcis) et le beurre de tournesol. Préférentiellement le ou les beurres selon l'invention sont choisis parmi le beurre de Murumuru, le beurre d'Ucuuba, le beurre de Shorea, le beurre d'Illipé, le beurre de Karité, le beurre de Cupuaçu et encore plus préférentiellement le beurre de Karité,.

Dans une variante préférée de l'invention la teneur pondérale en acides gras $C_{16}$ des triglycérides exprimée par rapport à la totalité des acides gras des triglycérides varie de 0 à 22 %, mieux de 0 à 15 %, encore mieux de 2 à 12 %.

La composition selon l'invention comprend un ou plusieurs beurres en quantité particulièrement comprise inclusivement entre 0,01 et 30 % en poids par rapport au poids total de la composition, plus particulièrement comprise inclusivement entre 0,1 et 20 % en poids, préférentiellement comprise inclusivement entre 0,5 et 10 % en poids, et plus préférentiellement comprise inclusivement entre 1 et 5 % en poids.

De préférence, les compositions de l'invention contiennent ou plusieurs corps gras liquides à la température ordinaire (25 °C) et à pression atmosphérique (760 mm de Hg ; soit 1,013.105 Pa), éventuellement associé(s) à un ou plusieurs corps gras non liquides.dans les mêmes conditions.

De préférence, le corps gras est choisi parmi

a) les beurres de préférence le beurre de karité,

b) les cires de préférence cires d'abeilles,

c) les alcools gras non liquides, particulièrement choisis parmi les alcools saturés ou insaturés, linéaires ou ramifiés, comportant de 8 à 30 atomes de carbone, préférentiellement en $C_{10}$-$C_{22}$, plus préférentiellement en $C_{14}$-$C_{20}$, mieux en $C_{16}$-$C_{18}$ tel que l'alcool cétylique et l'alcool stéarylique et leur mélange,

d) les esters d'acide gras et/ou d'alcools gras non liquides, notamment les esters solides issus d'acides gras en $C_9$-$C_{26}$ et d'alcools gras en $C_9$-$C_{26}$, en particulier les myristates d'alkyles tels que le myristate de cétyle, de mirystyle, de stéaryle ; le stéarate d'hexyle, plus particulièrement le myrystate de myristyle ;

e) les esters de monoalcools, dont un au moins de l'alcool ou de l'acide sont issus desdits esters est ramifié tels que les palmitates d'éthyle et d'isopropyle, les myristates d'alkyles tels que le myristate d'isopropyle, d'éthyle, le stéarate d'isocétyle, l'isononanoate d'éthyl-2-hexyle, le néopentanoate d'isodécyle, le néopentanoate d'isostéaryle, et les (iso)stéarates d'alkyles en $C_{10}$-$C_{22}$, de préférence en $C_{12}$-$C_{20}$ tels que l'isostéarate d'isopropyle ;

f) les polydialkylsiloxanes cycliques comportant de 3 à 7, de préférence de 4 à 5 atomes de silicium tel que l'octaméthylcyclotétrasiloxane, le décaméthylcyclopentasiloxane, le dodécaméthylcyclopentasiloxane, ou le cyclohexadiméthylsiloxane ainsi que leurs mélanges, de préférence cyclohexadiméthylsiloxane ;

g) les huiles d'origine végétale ou triglycérides synthétiques, telles que les triglycérides liquides d'acides gras comportant de 6 à 30 atomes de carbone comme les triglycérides des acides heptanoïque ou octanoïque ou encore, les huiles de tournesol, de maïs, de soja, de courge, de pépins de raisin, de sésame, de noisette, d'abricot, de macadamia, d'arara, de tournesol, de ricin, d'avocat, les triglycérides des acides caprylique / caprique, l'huile de jojoba, l'huile de beurre de karité, de préférence les triglycérides des acides caprylique / caprique.

Le ou les corps gras utilisés dans la composition selon la présente invention peuvent être présents dans la composition dans une quantité variant de 1 à 40 % en poids, de préférence en une quantité variant de 5 à 30 % en poids, et encore plus préférentiellement dans une quantité variant de 10 à 20 % en poids par rapport au poids total de la composition.

*Le ou les agents alcalins ;*

Selon un mode de réalisation particulier de l'invention, la composition de l'invention comprend un ou plusieurs agents alcalins (également appelés bases). Cet agent peut être choisi parmi les agents alcalins minéraux ou organiques ou hybrides ou leurs mélanges.
Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.
Selon un mode de réalisation avantageux de l'invention le ou les agents alcalins sont des amines organiques i.e ils contiennent au moins un groupe amino substitué ou non..
Le ou les agents alcalins organiques sont plus préférentiellement choisis parmi les amines organiques dont le $pK_b$ à 25 °C est inférieur à 12, et de préférence inférieur à 10, encore plus avantageusement inférieur à 6. **Il** est à noter qu'il s'agit du $pK_b$ correspondant à la fonction de basicité la plus élevée.
A titre de composés hybrides on peut mentionner les sels des amines citées précédemment avec des acides comme l'acide carbonique, l'acide chlorhydrique.
Le ou les agents alcalins organiques sont par exemple choisis parmi les alcanolamines, les éthylènediamines oxyéthylénées et/ou oxypropylénées, les acides aminés et les composés de formule (IV) suivante :

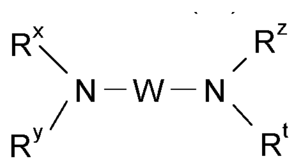
(IV)

Formule (IV) dans laquelle :

- W est un radical divalent alkylène en $C_1$-$C_6$ éventuellement substitué par un groupement hydroxyle ou un radical alkyle en $C_1$-$C_6$, et/ou éventuellement interrompu par un ou plusieurs hétéroatomes tels que l'oxygéne ou $NR^u$ ;
- $R^x$, $R^y$, $R^z$ $R^t$ et $R^u$, identiques ou différents, représentent un atome d'hydrogène, un radical alkyle en $C_1$-$C_6$ ou hydroxyalkyle en $C_1$-$C_6$, aminoalkyle en $C_1$-$C_6$.

On peut citer à titre d'exemple de telles amines, le 1,3 diaminopropane, le 1,3 diamino 2 propanol, la spermine, la spermidine.
Par alcanolamine on entend une amine organique comprenant une fonction amine primaire, secondaire ou tertiaire, et un ou plusieurs groupements alkyle, linéaires ou ramifiés, en $C_1$-$C_8$ porteurs d'un ou plusieurs radicaux hydroxyle.
Conviennent en particulier à la réalisation de l'invention les alcanolamines telles que les mono-, di- ou tri-alcanolamines, comprenant un à trois radicaux hydroxyalkyle, identiques ou non, en $C_1$-$C_4$.
Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthyl-aminométhane.
Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.
A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.
De manière avantageuse, les acides aminés sont des acides aminés basiques comprenant une fonction amine supplémentaire éventuellement incluse dans un cycle ou dans une fonction uréido. notamment choisis parmi l'histidine, la

lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la baleine.

L'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl] amino)ethane-1-sulfonique.

A titre de composés hybrides on peut mentionner en particulier utiliser le carbonate de guanidine ou le chlorhydrate de monoéthanolamine.

La composition de l'invention contient de préférence une ou plusieurs alcanolamines, et/ou un ou plusieurs acides aminés basiques, plus avantageusement, une ou plusieurs alcanolamines. Encore plus préférentiellement l'amine organique est la monoéthanolamine.

Selon un mode de réalisation particulier, la composition de l'invention comprend en tant qu'agent alcalin une ou plusieurs alcanolamines.

De préférence, l'alcanolamine est la triéthanolamine..

De manière avantageuse, la composition selon l'invention présente une teneur en agent(s) alcalin(s), allant de 0,01 à 10 % en poids, de préférence de 0,05 à 5 % en poids, mieux de 0,1 à 1 % en poids par rapport au poids de ladite composition.

*Le ou les polymères épaississants organiques :*

Selon un mode de réalisation particulier de l'invention la composition comprend un ou plusieurs polymères organiques épaississants.

Par « *polymère* épaississant » on entend un polymère qui introduit à 1% en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1 $s^{-1}$. Cette viscosité peut être mesurée à l'aide d'un viscosimètre cône/plan (Rhéomètre Haake R600 ou analogue). Les polymères épaississants peuvent être épaississants de la phase aqueuse et/ou de la phase grasse, préférentiellement de la phase aqueuse.

Par polymère épaississant « *organique »* on entend un polymère épaississant tel que défini précédemment qui est constitué de carbone, d'hydrogène, et éventuellement d'azote, d'oxygène, de soufre, d'halogènes tel que le fluor, le chlore, le brome ainsi que du phosphore, des métaux alcalins tels que le sodium, le potassium, ou métaux alcalino-terreux tels que le magnésium ou le calcium. Les polymères organiques selon l'invention ne comprennent pas de silicium.

Les polymères épaississants organiques selon l'invention peuvent être d'origine naturelle ou synthétique.

Les polymères épaississants peuvent être des polymères anioniques, cationiques, amphotères ou non ioniques, associatifs ou non.

Ils peuvent être épaississants des phases aqueuses ou huileuses.

A titre de polymères épaississants de phase aqueuse, on peut citer les polymères épaississants, associatifs ou non associatifs, de préférence non associatif, à motifs sucres.

Par motif *« sucre »* on entend au sens de la présente invention un motif issu d'un carbohydrate de formule $C_n(H_2O)_{n-1}$ ou $(CH_2O)_n$ pouvant être éventuellement modifié par substitution, et/ou par oxydation et/ou par déshydratation.

Les motifs sucre pouvant entrer dans la composition des polymères épaississants de l'invention sont de préférence issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose.

On peut notamment citer à titre de polymères épaississants de l'invention les gommes natives telles que :

a) les exsudats d'arbres ou d'arbustes dont :

- la gomme arabique (polymère ramifié de galactose, d'arabinose, de rhamnose et d'acide glucuronique) ;
- la gomme ghatti (polymère issu d'arabinose, de galactose, de mannose, de xylose et d'acide glucuronique) ;
- a gomme karaya (polymère issu d'acide galacturonique, de galactose, de rhamnose et d'acide glucuronique) ;
- la gomme tragacanthe (ou adragante) (polymère d'acide galacturonique, de galactose, de fucose, de xylose et d'arabinose) ;

b) les gommes issues d'algues dont :

- l'agar (polymère issu de galactose et d'anhydrogalactose) ;
- les alginates (polymères d'acide mannuronique et d'acide glucuronique) ;
- les carraghénanes et les furcelleranes (polymères de galactose sulfate et d'anhydrogalactose sulfate) ;

c) les gommes issus de semences ou tubercules dont :

- la gomme de guar (polymère de mannose et de galactose) ;
- la gomme de caroube (polymère de mannose et de galactose) ;
- la gomme de fenugrec (polymère de mannose et de galactose) ;
- la gomme de tamarin (polymère de galactose, de xylose et de glucose) ;
- la gomme de konjac (polymère de glucose et mannose) ;

d) les gommes microbiennes dont :

- la gomme de xanthane (polymère de glucose, de mannose acétate, de mannose/acide pyruvique et d'acide glucuronique) ;
- la gomme de gellane (polymère de glucose partiellement acylé, de rhamnose et d'acide glucuronique) ;
- la gomme de scléroglucane (polymère du glucose) ;

e) les extraits de plantes dont :

- la cellulose (polymère du glucose) ;
- l'amidon (polymère du glucose) et
- l'inuline.

Ces polymères peuvent être modifiés par voie physique ou chimique. A titre de traitement physique on peut citer notamment la température.
A titre de traitements chimiques on peut citer les réactions d'estérification, d'étherification, d'amidification, d'oxydation. Ces traitements permettent de conduire à des polymères qui peuvent être notamment non ioniques, anioniques ou amphotères.
De préférence ces traitements chimiques ou physiques sont appliqués sur les gommes de guar, les gommes de caroube, les amidons et les celluloses.
Les gommes de guar non-ioniques utilisables selon l'invention peuvent être modifiées par des groupements (poly)hydroxylakyle en $C_1$-$C_6$.
Parmi les groupements (poly)hydroxyalkyle en $C_1$-$C_6$, on peut mentionner à titre d'exemple, les groupements hydroxyméthyle, hydroxyéthyle, hydroxypropyle et hydroxybutyle.
Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.
Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.
Les molécules d'amidons utilisés dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.
Les amidons peuvent être modifiés par voie chimique ou physique : notamment par une ou plusieurs des réactions suivantes : prégélatinisation, oxydation, réticulation, estérification, éthérification, amidification, traitements thermiques.
On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).
Selon l'invention, on peut aussi utiliser des amidons amphotères, ces amidons amphotères comprennent un ou

plusieurs groupements anioniques et un ou plusieurs groupements cationiques. Les groupements anioniques et cationiques peuvent être liés au même site réactif de la molécule d'amidon ou à des sites réactifs différents, de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

Les polymères épaississants non associatifs de l'invention peuvent être des polymères cellulosiques ne comportant pas de chaînes grasse en $C_{10}$-$C_{30}$ dans leurs structure.

Par polymère *« cellulosique »,* on entend selon l'invention tout composé polysaccharidique possédant dans sa structure des enchaînements de résidus glucose unis par des liaisons $\beta$-1,4 ; outre les celluloses non substituées, les dérivés de celluloses peuvent être anioniques, cationiques, amphotères ou non-ioniques.

Ainsi, les polymères cellulosiques de l'invention peuvent être choisis parmi les celluloses non substituées y compris sous une forme microcristalline et les éthers de cellulose.

Parmi ces polymères cellulosiques, on distingue les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques sans chaine grasse en $C_{10}$-$C_{30}$ i.e. *« non associatifs »,* on peut citer les ($C_1$-$C_4$)alkylcelluloses telles que les méthylcelluloses et les éthylcelluloses (par exemple Ethocel standard 100 Premium de DOW CHEMICAL) ; les (poly)hydroxy($C_1$-$C_4$)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes (poly)hydroxy($C_1$-$C_4$)alkyl-($C_1$-$C_4$)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques sans chaine grasse, on peut citer les (poly)carboxy($C_1$-$C_4$)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques sans chaine grasse on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy($C_1$-$C_4$)alkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium. Les produits commercialisés répondant à cette définition sont plus particulièrement les produits vendus sous la dénomination "Celquat® L 200" et "Celquat® H 100" par la Société National Starch.

Selon un mode de réalisation particulier de l'invention le ou les polymères épaississant de l'invention sont issus de la (co)polymérisation de monomère acrylate $CH_2$=C(R')-COOR''' (VIa) et/ou de monomère acrylamide $CH_2$=C(R')-CO-N(R")-L-$Y^-$ $M^+$ (VIb) Formules (Via) et (VIb) dals lesquelles R', et R", identiques ou différents, représentant un atome d'hydrogène ou un groupe ($C_1$-$C_6$)alkyle tel que méthyle, de préférence hydrogène, R"' représente un métal alcalin, un métal alcalino-terreux, un atome d'hydrogène ou un groupe ($C_1$-$C_6$)alkyle éventuellement substitué notamment par un ou plusieurs groupes hydroxy, carboxy ou amino, de préférence R"' représente un atome d'hydrogène, L représentant un groupe hydrocarboné divalent, cyclique ou acylique, saturé ou insaturé, linéaire ou ramifié, éventuellement interrompu par un ou plusieurs hétéroatomes tels que O, N et comprenant de 1 à 20 atomes de carbones, de préférence de 1 à 6 atomes de carbones, de préférence L représente le groupe divalent -$[C(R')(R")]_p$- avec p représentant un entier compris entre 1 et 4, de préférence 2 et 3 tel que 2, R' et R" étant tels que définis précédemment, plus particulièrement L représente -C(R')(R")-$CH_2$- ou -$CH_2$-C(R')(R")- avec R' et R" tels que définis précédemment, de préférence R' et R" représentent un groupe ($C_1$-$C_4$)alkyle tel que méthyle ; $Y^-$représente un groupe anionique tel que carboxylate, phosphate, phosphonate, sulfonate, ou sulfate de préférence -$S(O)_2$-$O^-$, et $M^+$ étant un contre ion cationique de préférence métal alcalin tel que sodium, ledit copolymère pouvant se trouver en émulsion directe ou inverse, de préférence inverse. Plus préférentiellement le ou les polymères épaississant de l'invention sont issus de la copolymérisation de monomère acrylate $CH_2$=C(R')-COOH (VIa) et de monomère acrylamide $CH_2$=C(R')-CO-N(R")-L-$Y^-$ $M^+$ (VIb) tels que définis précédemment.

Parmi les polymères épaississants non associatifs sans motifs sucre utilisables, on peut citer les homopolymères ou copolymères d'acide acrylique ou méthacryliques réticulés, homopolymères réticulés d'acide 2-acrylamido-2-méthyl-propane sulfonique et leurs copolymères réticulés ou non d'acrylamide, les homopolymères d'acrylate d'ammonium ou les copolymères d'acrylate d'ammonium et d'acrylamide seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent être aussi des copolymères d'acide (méth)acryliques réticulés tels que le polymère vendu sous la dénomination AQUA SF1 par la société NOVEON.

Les polymères épaississants non associatifs peuvent être choisis parmi les homopolymères réticulés d'acide 2-acrylamido-2-méthylpropane sulfonique et leurs copolymères réticulés d'acrylamide.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom SIMULGEL 600 acrylamide/sodium acryloyldimethyltaurate copolymer isohexadecane et polysorbate 80 commercialisé par SEPPIC, MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

Parmi les polymères épaississants des phases aqueuses, on peut aussi citer les polymères associatifs non cellulosiques bien connus de l'homme de l'art et notamment de nature non ionique, anionique, cationique ou amphotère.

Il est rappelé que les *« polymères associatifs »* sont des polymères capables, dans un milieu aqueux, de s'associer réversiblement entre eux ou avec d'autres molécules.

Leur structure chimique comprend plus particulièrement au moins une zone hydrophile et au moins une zone hydrophobe.

Par *« groupement hydrophobe »,* on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs de type anionique, on peut citer :

- **(a)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci.

  Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth) acrylates d'alkyles inférieurs, de 2 à 50% en poids d'éther d'allyl à chaîne grasse, et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

  Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).

- **(b)** ceux comportant i) au moins un motif hydrophile de type acide carboxylique insaturé oléfinique, et ii) au moins un motif hydrophobe de type ester d'alkyl ($C_{10}$-$C_{30}$) d'acide carboxylique insaturé.

Des esters d'alkyles ($C_{10}$-$C_{30}$) d'acides carboxyliques insaturés utiles à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

On peut également citer le terpolymère acide acrylique/méthacrylate de lauryle/vinylpyrrolidone commercialisé sous l'appelation Acrylidone LM par la Société ISP.

- **(c)** les terpolymères d'anhydride maléique/α-oléfine en $C_{30}$-$C_{38}$/ maléate d'alkyle tel que le produit (copolymère anhydride maléique/α-oléfine en $C_{30}$-$C_{38}$/maléate d'isopropyle) vendu sous le nom PERFORMA V 1608® par la société NEWPHASE TECHNOLOGIES
- **(d)** les terpolymères acryliques comprenant :

  i) environ 20% à 70% en poids d'un acide carboxylique à insaturation α,β-monoéthylénique [A],
  ii) environ 20 à 80% en poids d'un monomère à insaturation α,β-monoéthylénique non-tensio-actif différent de [A],
  iii) environ 0,5 à 60% en poids d'un mono-uréthane non-ionique qui est le produit de réaction d'un tensio-actif monohydrique avec un monoisocyanate à insaturation monoéthylénique,

  tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- **(e)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en $C_1$-$C_4$. A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

- ***(f)*** Les polymères amphiphiles comportant au moins un monomère à insaturation éthylènique à groupement sulfonique, sous forme libre ou partiellement ou totalement neutralisée et comprenant au moins une partie hydrophobe. Ces polymères peuvent être réticulés ou non-réticulés. Ils sont de préférence réticulés.

Les monomères à insaturation éthylènique à groupement sulfonique sont choisis notamment parmi l'acide vinylsulfonique, l'acide styrènesulfonique, les acides (méth)acrylamido($C_1$-$C_{22}$)alkylsulfoniques, les acides *N*-($C_1$-$C_{22}$) alkyl(méth)-acrylamido-($C_1$-$C_{22}$)alkylsulfoniques comme l'acide undécyl-acrylamido-méthane-sulfonique ainsi que leurs formes partiellement ou totalement neutralisées.

Plus préférentiellement, on utilisera les acides (méth)acrylamido($C_1$-$C_{22}$) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères de cette famille peuvent notamment être choisis parmi les polymères amphiphiles statistiques d'AMPS modifiés par réaction avec une n-monoalkylamine ou une di-n-alkylamine en $C_6$-$C_{22}$, et tels que ceux décrits dans la demande de brevet WO 00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Les polymères préférés de cette famille sont choisis parmi les copolymères amphiphiles d'AMPS et d'au moins un monomère hydrophobe à insaturation éthylénique.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :

- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules, Vol. 33, N° 10 (2000), 3694-3704 » ;
- « Solution properties of miscelle networks formed by non-ionic moieties covalently bound to an polyelectrolyte : salt effects on rheological behavior - Langmuir, , Vol. 16, N°12, (2000) 5324-5332 » ;
- « Stimuli responsive amphiphilic copolymers of sodium 2-(acrylamido)-2-methylpropanesulfonate and associative macromonomers - Polym. Preprint, Div. Polym. Chem., 40(2), (1999), 220-221».

Parmi ces polymères, on peut citer :

- les copolymères réticulés ou non réticulés, neutralisés ou non, comportant de 15 à 60 % en poids de motifs AMPS et de 40 à 85 % en poids de motifs ($C_8$-$C_{16}$)alkyl(méth)acrylamide ou de motifs ($C_8$-$C_{16}$)alkyl(méth)acrylate par rapport au polymère, tels que ceux décrits dans la demande EP-A750 899 ;
- les terpolymères comportant de 10 à 90 % en mole de motifs acrylamide, de 0,1 à 10% en mole de motifs AMPS et de 5 à 80% en mole de motifs n-($C_6$-$C_{18}$)alkylacrylamide, tels que ceux décrits dans le brevet US-5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.
Parmi les polymères associatifs cationiques on peut citer :

- **(I)** Les polyuréthanes associatifs cationiques ;
- ***(II)*** Le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :

* un méthacrylate de di(alkyl en $C_1$-$C_4$) amino(alkyle en $C_1$-$C_6$),
* un ou plusieurs esters d'alkyle en $C_1$-$C_{30}$ et de l'acide (méth)acrylique,
* un méthacrylate d'alkyle en $C_{10}$-$C_{30}$ polyéthoxylé (20-25 moles de motif oxyde d'éthylène),
* un allyl éther de polyéthylèneglycol/polypropylèneglycol 30/5,
* un méthacrylate d'hydroxy(alkyle en $C_2$-$C_6$), et
* un diméthacrylate d'éthylèneglycol.

- ***(III)*** les (poly)hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci. Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle. On peut indiquer comme exemples d'alkylhydroxyéthylcelluloses quaternisées à chaînes grasses en $C_8$-$C_{30}$, telles que les produits QUATRISOFT LM 200®, QUATRISOFT LM-X 529-18-A®, QUATRISOFT LM-X 529-18-B® (alkyle en $C_{12}$) et QUATRISOFT LM-X 529-8® (alkyle en $C_{18}$) vendus par la société AQUALON, les produits CRODACEL QM®, CRODACEL QL® (alkyle en $C_{12}$) et CRODACEL QS® (alkyle en $C_{18}$) vendus par la société CRODA et le produit SOFTCAT SL 100® vendu par la société AQUALON.
- ***(IV)*** les Polymères polyvinyllactames cationiques.

De tels polymères sont par exemple décrits dans la demande de brevet WO-00/68282.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthyl-méthacrylamidopropylammonium, les terpolymères vinylpyrrolidone / diméthylamino-propylméthacrylamide / tosylate ou chlorure de lauryldiméthylméthacrylamido-propylammonium.

Les polymères associatifs amphotères sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles % et plus particulièrement encore 1,5 à 6 moles %, par rapport au nombre total de moles de monomères.

Des polymères associatifs amphotères selon l'invention sont par exemple décrits et préparés dans la demande de brevet WO 9844012.

Parmi les polymères associatifs amphotères selon l'invention, on préfère les terpolymères acide acrylique / chlorure de (méth)acrylamidopropyl triméthyl ammonium / méthacrylate de stéaryle.

Les polymères associatifs de type non ionique utilisables selon l'invention sont choisis de préférence parmi :

(a) les copolymères de vinyl pyrrolidone et de monomères hydrophobes à chaîne grasse dont on peut citer à titre d'exemple :

- les produits ANTARON V216® ou GANEX V216® (copolymère vinylpyrrolidone / hexadécène) vendu par la société I.S.P.
- les produits ANTARON V220® ou GANEX V220® (copolymère vinylpyrrolidone / eicosène) vendu par la société I.S.P.

(b) les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.

(c) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

(d) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

(e) les polymères à squelette aminoplaste éther possédant au moins une chaîne grasse, tels que les composés PURE THIX® proposés par la société SUD-CHEMIE.

(f) les celluloses ou leurs dérivés, modifiés par des groupements comportant au moins une chaîne grasse tels que des groupes alkyls, arylalkyls, alkylaryls ou leurs mélanges où les groupes alkyls sont en $C_8$- et en particulier :

* les alkylhydroxyéthylcelluloses non-ioniques telles que les produits NATROSOL PLUS GRADE 330 CS et POLYSURF 67 (alkyle en $C_{16}$) vendus par la société AQUALON
* les nonoxynylhydroxyéthylcelluloses non-ioniques tels que le produit AMERCELL HM-1500 vendu par la société AMERCHOL ;
* les alkylcelluloses non-ioniques telles que le produit BERMOCOLL EHM 100 vendu par la société BEROL NOBEL ;

(g) les dérivés de guar associatifs comme les hydroxypropylguars modifiés par une chaîne grasse tel que le produit ESAFLOR HM 22 (modifié par une chaîne alkyle en $C_{22}$) vendu par la société LAMBERTI ; le produit MIRACARE XC 95-3 (modifié par une chaîne alkyle en $C_{14}$) et le produit RE 205-146 (modifié par une chaîne alkyle en $C_{20}$) vendus par RHODIA CHIMIE ;

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes

polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208, 204 ou 212, ainsi que l'Acrysol RM 184®.

On peut également citer le produit ELFACOS T210® à chaîne alkyle en $C_{12}$-$C_{14}$ et le produit ELFACOS T212® à chaîne alkyle en $C_{18}$ de chez AKZO.

Le produit DW 1206B® de chez ROHM & HAAS à chaîne alkyle en $C_{20}$ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le RHEOLATE® 255, le RHEOLATE® 278 et le RHEOLATE® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations ACULYN 46® et ACULYN 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut aussi utiliser des polymères épaississants des phases grasses.

De préférence, les polymères structurant la phase huileuse via des interactions physiques sont choisis parmi les polyamides, les polyamides siliconés, les mono- ou poly-alkylesters de saccharide ou polysaccharide, les dérivés amides d'aminoacides N-acylés, copolymères comprenant une séquence alkylène ou styrène, ces copolymères pouvant être des polymères di-blocs, tri-bloc, multi-bloc, radial-bloc encore appelés copolymères en étoile, ou encore des polymères en peigne.

1) Les polymères portant dans le squelette au moins une séquence cristallisable Il s'agit encore de polymères solubles ou dispersibles dans l'huile ou phase huileuse par chauffage au-dessus de leur point de fusion pF. Ces polymères sont notamment des copolymères séquencés constitués d'au moins 2 séquences de nature chimique différente dont l'une est cristallisable.

A titre de polymères portant dans le squelette au moins une séquence cristallisable convenant à la mise en oeuvre de l'invention, on peut mentionner :

i). Les polymères définis dans le document US-A-5,156,911 ;
ii). Les copolymères séquencés d'oléfine ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène 2), 5-méthylnorbornène, 5-éthylnorbornène, 5,6-diméthyl-norbornène, 5,5,6-triméthyl norbornène, 5-éthylidène-norbornène, 5-phénylnorbornène,
  5-benzylnorbornène, 5-vinylnorbornène, 1,4,5,8-diméthano-1,2,3,4,4a,5,8a-octahydronaphtalène, dicyclopentadiène, et leurs mélanges ;
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges.

Ces copolymères séquencés peuvent être en particulier les copolymères (éthylène/norbornène) blocs et les terpolymères blocs (éthylène/propylène/ éthylidène-norbornène).

On peut aussi utiliser ceux résultants de la copolymérisation séquencée d'au moins 2 $\alpha$-oléfines en $C_2$-$C_{16}$ et mieux en $C_2$-$C_{12}$, tels que ceux cités précédemment et en particulier les bipolymères séquencés d'éthylène et d'1-octène.
Les copolymères présentant au moins une séquence cristallisable, le reste du copolymère étant amorphe (à température ambiante). Ces copolymères peuvent, en outre, présenter deux séquences cristallisables de nature chimique différente.
Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :

a) les copolymères séquencés poly($\delta$-caprolactone)-b-poly(butadiène), utilisés de préférence hydrogénés, tels que ceux décrits dans l'article « Melting behavior of poly($\delta$-caprolactone)-block-polybutadiène copolymers » de S. Nojima, Macromolécules, 32, 3727-3734 (1999).
b) les copolymères séquencés poly(butylènetéréphtalate)-b-poly(isoprène) hydrogénés séquencés ou multiséquencés, cités dans l'article « Study of morphological and mechanical properties of PP/PBT » de B. Boutevin et al., Polymer Bulletin, 34, 117-123 (1995).
c) les copolymères séquencés poly(éthylène)-b-copoly(éthylène/propylène) cités dans les articles « Morphology of semi-crystalline block copolymers of ethylene-(ethylene-alt-propylene) » de P. Rangarajan et al., Macromolecules, 26, 4640-4645 (1993), et « Polymer agregates with crystalline cores : the system poly(ethylene)- poly(ethylene-propylene) », P. Richter et al., Macromolécules, 30, 1053-1068 25 (1997).
d) les copolymères séquencés poly(éthylène)-b-poly(éthyléthylène) cités dans l'article général « Cristallization in block copolymers » de I.W. Hamley, Advances in Polymer Science, vol 148, 113-137 (1999).

Les polymères semi-cristallins utilisables dans le cadre de l'invention peuvent être non réticulés ou réticulés en partie, du moment que le taux de réticulation ne gêne pas leur dissolution ou dispersion dans la phase huileuse liquide par chauffage au dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.
De préférence, les polymères semi-cristallins convenant à l'invention sont non réticulés.
A titre d'exemple particulier de polymère semi-cristallin utilisable dans la composition selon l'invention, on peut citer les produits Intelimer® de la société Landec décrits dans la brochure « Intelimer® polymers ». Ces polymères sont sous forme solide à température ambiante (25 °C). Ils sont porteurs de chaînes latérales cristallisables et présentent le monomère. On peut citer notamment le « Landec IP22® », ayant une température de fusion pF de 56 °C, qui est un produit visqueux à température ambiante, imperméable, non-collant.
On peut aussi utiliser les polymères semi-cristallins décrits dans les exemples 3, 4, 5, 7, 9 du document US-A-5,156,911, résultant de la copolymérisation d'acide acrylique et d'alkyl(méth)acrylate en $C_5$ à $C_{16}$ comme ceux résultant de la copolymérisation :

- d'acide acrylique, d'hexadécylacrylate et d'isodécylacrylate dans un rapport 1/16/3,
- d'acide acrylique et de pentadécylacrylate dans un rapport 1/19,
- d'acide acrylique, d'hexadécylacrylate, d'éthylacrylate dans un rapport 2,5/76,5/20,
- d'acide acrylique, d'hexadécylacrylate et de méthylacrylate dans un rapport 5/85/10,
- d'acide acrylique, d'octadécylméthacrylate dans un rapport 2,5 / 97,5.

On peut aussi utiliser le polymère « Structure O » commercialisé par la société National Starch, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.
On peut encore utiliser les polymères semi-cristallins obtenus par copolymérisation d'acrylate de stéaryle et

d'acide acrylique ou de NVP ou par copolymérisation de l'acrylate de béhényle et de l'acide acrylique ou de NVP, tels que décrits dans le document US-A-5,519,063 ou EP-A- 0 550 745.

Selon une variante particulière de réalisation, les polymères semi-cristallins convenant à la mise en œuvre de la présente invention sont notamment les acrylates alkylés, parmi lesquels on peut mentionner les copolymères de LANDEC :

- Doresco IPA 13-1® : poly acrylate de stéaryle, pf de 49 °C et PM de 145000;
- Doresco IPA 13-3® : poly acrylate/ acide méthacrylique, pf de 65 °C et PM de 114000;
- Doresco IPA 13-4® : poly acrylate/ vinyl pirrolidone, pf de 44 °C et PM de 387000;
- Doresco IPA13-5® : poly acrylate / methacrylate d'hydroxyethyle, pf de 47 °C et PM de 397600;
- Doresco IPA 13-6® : poly acrylate de béhényle, pf de 66 °C.

2) Les polyamides non siliconés

Les polyamides particuliers utilisés dans la composition selon la présente invention sont de préférence ceux décrits dans le document US-A-5,783,657 de la Société UNION CAMP. La partie de US-A-5,783,657 consacrée à ces polymères est incorporée par référence.

Chacun de ces polyamides satisfait notamment à la formule (V) suivante :

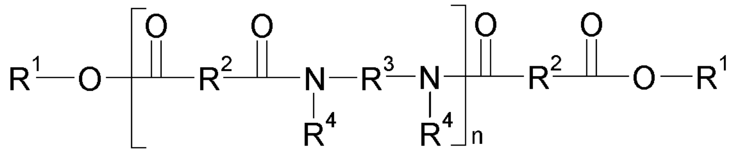

(V)

Formule (V) dans laquelle :

- n désigne un nombre entier de motifs amide tel que le nombre de groupes ester représente de 10 % à 50 % du nombre total des groupes ester et amide ;
- $R^1$ est à chaque occurrence indépendamment un groupe alkyle ou alcényle ayant au moins 4 atomes de carbone et notamment de 4 à 24 atomes de carbone
- $R^2$ représente à chaque occurrence indépendamment un groupe hydrocarboné en $C_4$ à $C_{55}$ à condition que 50 % au moins des groupes $R_2$ représentent un groupe hydrocarboné en $C_{30}$ à $C_{55}$ ;
- $R^3$ représente à chaque occurrence indépendamment un groupe organique pourvu d'au moins 2 atomes de carbone, d'atomes d'hydrogène et optionnellement d'un ou plusieurs atomes d'oxygène ou d'azote ; et
- $R^4$ représente à chaque occurrence indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-$R_3$, avec au moins 50 % des $R_4$ représentant un atome d'hydrogène.

En particulier, les groupes ester de ce polyamide représentent de 15 à 40 % du nombre total des groupes ester et amide et au mieux de 20 à 35 %. De plus, n représente avantageusement un nombre entier allant de 1 à 10, et mieux de 1 à 5, bornes incluses.

De préférence, $R^1$ est un groupe alkyle en $C_{12}$ à $C_{22}$ et de préférence en $C_{16}$ à $C_{22}$. Avantageusement, $R^2$ peut être un groupe hydrocarboné (alkylène) en $C_{10}$ à $C_{42}$. De préférence, 50 % au moins et mieux 75 % au moins des $R^2$ sont des groupes ayant de 30 à 42 atomes de carbone. Les autres $R^2$ sont des groupes hydrogénés en $C_4$ à $C_{19}$ et de préférence en $C_4$ à $C_{12}$. De préférence, $R^3$ représente un groupe hydrocarboné en $C_2$ à $C_{36}$ ou un groupe polyoxyalkyléné et $R^4$ représente un atome d'hydrogène. De préférence, $R^3$ représente un groupe hydrocarboné en $C_2$ à $C_{12}$. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non. L'épaississement de la phase grasse liquide peut être obtenue à l'aide d'un ou plusieurs polyamides définis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Comme polyamide structurant utilisable dans l'invention, on peut encore citer les résines polyamides résultant de la condensation d'un acide di-carboxylique aliphatique et d'une diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp., sous la marque Onamid® notamment Onamid S ou C. Ces résines ont une

masse moléculaire moyenne en poids allant de 6000 et 9000. Pour plus d'information sur ces polyamides, on peut se référer aux documents US-A-3,645,705 et US-A-3,148,125. Plus spécialement, on utilise les Versamid® 30 ou 744.

On peut aussi utiliser les polyamides vendus ou fabriqués par la société Arizona sous les références Uni-Rez (2658, 2931, 2970, 2621,2613, 2624, 2665, 1554, 2623, 2662) et le produit vendu sous la référence Macromelt 6212 par la société Henkel. Pour plus d'information sur ces polyamides, on peut se référer au document USA-5500209.

A titre d'exemple de polyamides structurant utilisables dans la composition selon l'invention, on peut encore citer les produits commerciaux vendus ou fabriqués par la société Arizona Chemical sous les noms Uniclear 80 et Uniclear 100. Ils sont vendus respectivement sous forme de gel à 80 % (en matière active) et à 100% (en matière active) dans une huile minérale. Ils ont un point de ramollissement de 88 à 105 °C. Ces produits commerciaux sont un mélange de copolymère d'un diacide en C36 condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

**2)** Les mono- ou polyalkylesters de saccharide ou polysaccharide

Parmi les mono ou polyalkylesters de saccharide ou de polysaccharide convenant à la mise en œuvre de l'invention, on peut mentionner les alkyles ou polyalkylesters de dextrine ou d'inuline.

Il peut s'agir notamment d'un mono-ou poly-ester de dextrine et d'au moins un acide gras et notamment répondant à la formule (VI) suivante :

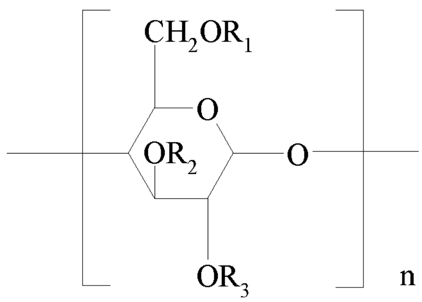

(VI)

Formule (VI) dans laquelle :

- n est un entier allant de 3 à 200, notamment allant de 20 à 150, et en particulier allant de 25 à 50,
- $R_1$, $R_2$ et $R_3$, identiques ou différents, sont choisis parmi l'hydrogène ou un groupement acyle (R-C(O)-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux $R_1$, $R_2$ ou $R_3$ est différent de l'hydrogène.

En particulier, $R_1$, $R_2$ et $R_3$ peuvent représenter l'hydrogène ou un groupement acyle (R-C(O)-) dans lequel R est un radical hydrocarboné tel que défini précédemment, sous réserve qu'au moins deux desdits radicaux $R_1$, $R_2$ ou $R_3$ sont identiques et différents de l'hydrogène.

L'ensemble des radicaux $R_1$, $R_2$ et $R_3$ peut figurer un groupement acyle (R-C(O)) identique ou différent, et notamment identique.

En particulier, n précédemment exposé varie avantageusement de 25 à 50, notamment est égal à 38 dans la formule générale de l'ester de saccharide utilisable dans la présente invention.

Notamment lorsque les radicaux $R_1$, $R_2$ et/ou $R_3$, identiques ou différents figurent un groupement acyle (R-C(O)), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.
Des esters de dextrine, en particulier des palmitates de dextrine, sont disponibles commercialement sous la dénomination RHEOPEARL TL ou RHEOPEARL KL de la société Chiba Flour.

3) les dérivés amides d'aminoacides N-acylés

Les amides d'aminoacides N-acylés utilisables sont par exemple les diamides de l'association d'un acide N-acylamine avec des amines comprenant de 1 à 22 atomes de carbone tels que ceux décrits dans le document FR 2 281 162. Ce sont par exemple les dérivés amides d'acide alcoyle glutamique tels que le dibutylamide d'acide laurylglutamique, commercialisé par la société Ajinomoto sous le nom « Gelling agent GP-1 » ou encore le dibutylamide d'acide 2-éthylhexanoyl glutamique commercialisé par la société Ajinomoto sous la dénomination de « Gelling agent GA-01 ».

**4)** Les copolymères comprenant une séquence alkylène ou styrène

Les copolymères peuvent avoir une structure en peigne ou bloc de type di-bloc, tri-bloc, multi-bloc et/ou radial ou étoilé et comporter au moins deux segments incompatibles du point de vue thermodynamique.
L'agent structurant peut comprendre, par exemple, un bloc segment styrène comme décrit dans les demandes EP 0 497 144, WO98/42298, US 6 225 690, US 6 174 968, US 6 225 390, un segment éthylène/butylène, un segment éthylène/propylène comme décrit dans les demandes US 6 225 690, US 6 174 968, US 6 225 390, un segment butadiène, un segment isoprène, un segment polyvinyl comme par exemple poly(meth)acrylate d'alkyle,ou polyvinyl alcool ou polyacétate de vinyl, un segment siliconé comme décrit dans les demandes US 5 468 477 et US 5 725 882 ou une combinaison de ces segments.
Un copolymère di-bloc est habituellement défini comme étant de type A-B dans lequel un segment dur (A) est suivi par un segment souple (B).
Un copolymère tri-bloc est habituellement défini comme étant de type A-B-A ou comme un rapport d'un segment dur, d'un segment souple et d'un segment dur.
Un copolymère multi-bloc ou radial ou étoilé peut comporter n'importe quel type de combinaison de segments durs et de segments souples, sous réserve que les caractéristiques des segments durs et des segments souples soient conservées.
A titre d'exemple de segments durs de copolymère bloc, il peut être fait mention du styrène, et à titre d'exemple de segments souples de copolymère bloc, il peut être fait mention de l'éthylène, du propylène, du butylène, et d'une combinaison de ceux-ci.
Les copolymères tri-bloc, et notamment ceux de type polystyrène/polyisoprène ou polystyrène/polybutadiène, convenant à la mise en œuvre de l'invention peuvent être ceux commercialisés sous la référence LUVITOL HSB par la société BASF. Il peut également être fait mention des copolymères tri-bloc de type polystyrène/copoly(éthylène-propylène) ou polystyrène/ copoly(éthylène - butylène), tels que ceux commercialisés sous la référence KRATON par la société SHELL CHEMICAL CO, ou sous la référence GELLED PERMETHYL 99 A par la société PENRECO. De tels copolymères tri-blocs sont particulièrement préférés selon l'invention.
A titre d'exemple, encore, de copolymères bloc susceptibles de convenir à la mise en œuvre de la présente invention, il peut également être fait mention des copolymères blocs commercialisés sous la référence VERSAGEL par la société PENRECO, ceux commercialisés sous la référence KRATON par la société SHELL et ceux commercialisés sous la référence GEL BASE par la société BROOKS INDUSTRIES.
Parmi les polymères épaississants pour phase grasse les polymères portant dans le squelette au moins une séquence cristallisable sont préférés.
Les polymères épaississants pour phase aqueuse ou phase grasse peuvent être utilisés seuls ou en mélanges en toutes proportions.
De préférence les épaississants sont des épaississants de phase aqueuse.
De préférence, les polymères des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 s-1.
Selon un mode de réalisation préféré de l'invention le ou les polymères épaississant de l'invention sont non associatifs et préférentiellement issus de la (co)polymérisation de monomère acrylate $CH_2=C(R')-COOR'''$ (VIa) et/ou de monomère acrylamide $CH_2=C(R')-CO-N(R'')-L-Y^- M^+$ (VIb) Formules (Via) et (VIb) dans lesquelles R', et R", identiques ou différents, représentant un atome d'hydrogène ou un groupe $(C_1-C_6)$alkyle tel que méthyle, de préférence hydrogène, R'" représente un métal alcalin, un métal alcalino-terreux, un atome d'hydrogène ou un groupe $(C_1-C_6)$alkyle éventuel-

lement substitué notamment par un ou plusieurs groupes hydroxy, carboxy ou amino, L représentant un groupe hydrocarboné divalent, cyclique ou acylique, saturé ou insaturé, linéaire ou ramifié, éventuellement interrompu et/ou substitué par un ou plusieurs hétéroatomes tels que O, N et comprenant de 1 à 20 atomes de carbones, de préférence de 1 à 6 atomes de carbones, de préférence L représente le groupe divalent $-[C(R')(R'')]_p-$ avec p représentant un entier compris entre 1 et 4, de préférence 2 et 3 tel que 2, R' et R" étant tels que définis précédemment, plus particulièrement L représente $-C(R')(R'')-CH_2-$ ou $-CH_2-C(R')(R'')-$ avec R' et R" tels que définis précédemment, de préférence R' et R" représentent un groupe $(C_1-C_4)$alkyle tel que méthyle ; Y-représente un groupe anionique tel que carboxylate, phosphate, phosphonate, sulfonate, ou sulfate de préférence $-S(O)_2-O^-$, et M+ étant un contre ion cationique de préférence métal alcalin tel que sodium, ledit copolymère pouvant se trouver en émulsion directe ou inverse, de préférence inverse. Plus préférentiellement le ou les polymères épaississant de l'invention sont issus de la copolymérisation de monomère acrylate $CH_2=C(R')-COOH$ (VIa) et de monomère acrylamide $CH_2=C(R')-CO-N(R'')-L-Y^-\ M^+$ (VIb) tels que définis précédemment. Parmi les polymères épaississants on peut citer les ; de préférence les polymères organiques épaississants sont choisis parmi les copolymères d'acide acrylique ou méthacryliques réticulés ou non, homopolymères réticulés ou non d'acide 2-acrylamido-2-méthyl-propane sulfonique et leurs copolymères réticulés ou non d'acrylamide, les homopolymères d'acrylate d'ammonium ou les copolymères d'acrylate d'ammonium et d'acrylamide seuls ou en mélanges.

Selon une variante avantageuse la composition de l'invention comprend un ou plusieurs polymères épaississants associatif ou non associatifs, de préférence non associatif, à motifs sucres en particulier issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose, de préférence de galactose d'anhydrogalactose, de préférence l'agar.

De préférence le ou les polymères épaississants organiques est ou sont présents dans la composition selon l'invention dans une teneur allant de 0,01 à 10 % en poids plus préférentiellement de 0,1 à 5 % en poids par rapport au poids total de la composition.

Selon un mode de réalisation particulier le ratio pondéral corps gras / tensioactif(s) est compris inclusivement entre 5 et 20, de préférence entre 8 et 15, encore plus préférentiellement entre 10 et 13 tel que 11,8.

Selon un mode de réalisation de l'invention le ratio pondéral en corps gras / somme de tensioactif(s) et polymère(s) [tensioactif(s) + polymère] est compris inclusivement entre 0,8 et 10, particulièrement entre 1 et 5, plus particulièrement entre 1,5 et 2,5, tel que 1,9.

De préférence, la composition comprend la i) 4-(3-éthoxy-4-hydroxyphényl)butan-2-one ainsi que ses sels d'acide ou de base, organique ou minéral, et ses solvates tels que les hydrates en une teneur allant de 0,02 à 2 % en poids, par rapport au poids total de la composition, préférentiellement allant de 0,03 à 1 % en poids, et mieux allant de 0,04 à 0,8 % en poids.

L'invention a aussi pour objet une composition comprenant, dans un milieu physiologiquement acceptable, le mélange antimicrobien décrit précédemment.

On entend par milieu physiologiquement acceptable, un milieu compatible avec les matières kératiniques d'êtres humains tels que la peau, le cuir chevelu, les ongles et les fibres kératiniques telles que les cheveux.

Ledit milieu peut comprendre un ou plusieurs ingrédients additionnels, distinct des ingrédients i) et ii).

La composition peut comprendre un ou plusieurs ingrédients additionnels ou adjuvants choisis parmi les gélifiants différents des polymères épaississants organiques tels que définis précédemment, les polymères non épaississants non anioniques, cationiques, non ioniques, zwitterioniques, naturels ou synthétiques, filmogènes ou non, les matières colorantes tels que les pigments organiques ou minéraux, les parfums, les charges, les filtres UV, les extraits végétaux, les actifs cosmétiques et dermatologiques, et les sels.

La composition selon l'invention peut se présenter sous forme d'émulsions huile dans- eau (H/E) ou eau-dans-huile (E/H) ou multiple (triple : E/H/E ou H/E/H), de solutions huileuses, de gels huileux, de solutions aqueuses, de gels aqueux, de compositions solides. La composition de l'invention est préparée selon les méthodes usuelles.

La composition selon l'invention de préférence comprend de l'eau i.e. est aqueuse. Selon un mode de réalisation de l'invention la composition comprend une phase aqueuse et une phase organique ou huileuse. De préférence la composition de l'invention est une émulsion directe de type H/E.

Selon un autre mode de réalisation particulier de l'invention la composition est aqueuse et ne comprend pas de tensioactifs.

Les compositions selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick ou de poudre compacte.

La composition selon l'invention peut notamment se présenter sous la forme :

- d'un produit de maquillage, notamment de la peau du visage, du corps ou des lèvres ou des cils ;
- d'un gel ou lotion après-rasage; de produit de rasage ;

- d'un déodorant (stick, roll-on, aérosol)
- d'une crème dépilatoire;
- sous la forme d'une composition d'hygiène corporelle telle qu'un gel douche ou un shampooing;
- d'une composition pharmaceutique;
- d'une composition solide telle qu'un savon ou un pain de nettoyage;
- d'une composition aérosol comprenant également un agent propulseur sous pression;
- d'une lotion de mise en plis, d'une crème ou d'un gel coiffant, d'une composition de teinture, d'une composition de permanente, d'une lotion ou d'un gel antichute, d'un après shampooing ;
- d'une composition de soin ou de nettoyage de la peau.

*Le Procédé de préparation de la composition* :

L'invention a aussi pour objet un procédé de préparation d'une composition, notamment cosmétique ou pharmaceutique ou alimentaire, comprenant une étape de mélange de la i) 4-(3-éthoxy-4-hydroxyphényl)butan-2-one, un de ses sels de bases ou solvates, de la ii) 4-hydroxyacétophénone un de ses sels de bases ou solvates, et éventuellement d'un ou plusieurs ingrédients additionnels ou adjuvants, notamment cosmétiques ou pharmaceutiques ou alimentaires, tels que ceux décrits précédemment, et éventuellement de l'eau, et un ou plusieurs solvants organiques.

*Activité antimicrobienne et leur utilisation* :

L'activité antimicrobienne notamment de synergie du mélange i) et ii) selon l'invention sur les champignons, en particulier sur l'espèce *candida albicans* présente un intérêt dans le traitement de l'eau. En effet, les champignons représentent une des sources de contamination de l'eau comme mentionné dans l'article « Fungal Contaminants in drinking water regulation ?,A tale of ecology, exposure, purification and clinical relevance » Int. J. Environ. Res. Public Health 2017, 14, 636.

La présente invention concerne également l'utilisation du mélange antimicrobien dans le traitement de l'eau, où ladite eau est choisie parmi les eaux domestiques ou industrielles, les eaux des milieux aquatiques, les eaux de piscine/spa, et les eaux de système de climatisation.

On entend par « traitement de l'eau » un traitement consistant à ajouter une substance à un échantillon d'eau à traiter ou à un flux d'eau à traiter continu ou discontinu (type batch) en vue soit de prévenir la contamination de l'eau par un agent contaminant soit de décontaminer partiellement ou totalement ladite eau à traiter dudit agent contaminant.

De préférence, le traitement de l'eau réalisé dans le cadre de la présente invention consiste à ajouter une substance à un échantillon d'eau à traiter ou à un flux d'eau à traiter continu ou discontinu afin de décontaminer partiellement ou totalement ladite eau à traiter d'un agent contaminant.

L'agent contaminant peut être un microorganisme, en particulier une bactérie et/ou un champignon.

Encore plus préférentiellement, ledit traitement de l'eau est un traitement de l'eau contaminée par un ou plusieurs microorganismes, de préférence par des bactéries Gram +/- ou champignons de l'espèce *Enterococcus faecalis, Candida albicans, Pseudomonas aeruginosa.*

On entend par « eaux des milieux aquatiques » les eaux des lacs, rivières, étangs, fleuves, zones de baignades de mer ou d'océan, les eaux souterraines telles que les eaux des puits et nappes phréatiques, les eaux d'aquariums.

Au sens de la présente invention les « eaux domestiques ou industrielles » comprennent les eaux usées avant leur traitement en station d'épuration, les eaux en cours de traitement en station d'épuration, les eaux avant leur traitement en station de potabilisation, les eaux en cours de traitement en station de potabilisation, ainsi que les eaux circulant dans les réseaux urbains potables ou non potables comme par exemple les eaux circulant dans les canalisations.

La présente invention se rapporte également à un procédé de traitement d'eau comprenant au moins une étape de mise en contact d'un échantillon d'eau à traiter ou d'un flux d'eau à traiter continu ou discontinu, ladite eau à traiter étant choisie parmi les eaux domestiques ou industrielles, les eaux des milieux aquatiques, les eaux de piscine/spa, et les eaux de système de climatisation, avec le mélange antimicrobien selon l'invention.

De préférence, ladite étape de mise en contact de l'eau à traiter avec le mélange antimicrobien selon l'invention peut notamment être réalisée par injection sous forme liquide dudit composé, par passage sur un filtre ou une cartouche filtrante comprenant ledit composé, ou encore par administration sous forme solide dudit composé notamment sous la forme de granulés, de galets ou encore de pastilles.

La i) 4-(3-éthoxy-4-hydroxyphényl)butan-2-one ainsi que ses sels d'acide ou de base, organique ou minéral, et ses solvates tels que les hydrates peut être employée à raison d'au moins 0,06 % en poids, de préférence au moins 0,1 % en poids, encore mieux au moins 0,5 % en poids par rapport au poids total d'eau à traiter. Dans un mode de réalisation particulier, les composés de formule (I) ou (I') ou ses solvates tels que les hydrates, peuvent être employés à raison d'au moins 1 % en poids par rapport au poids total d'eau à traiter.

La i) 4-(3-éthoxy-4-hydroxyphényl)butan-2-one ainsi que ses sels d'acide ou de base, organique ou minéral, et

ses solvates tels que les hydrates peut être utilisée en une concentration allant de 0,06 % à 10 % en poids, de préférence de 0,1 % à 5 % en poids, encore mieux de 0,5 % à 2 % en poids par rapport au poids total d'eau à traiter. Dans un mode de réalisation préféré, la 4-(3-éthoxy-4-hydroxyphényl)butan-2-one peuvent être utilisé en une concentration allant de 0,1 % à 1 % en poids par rapport au poids total d'eau à traiter.

Le solvant peut être utilisé en une teneur allant de 0,05 % à 10 % en poids, par rapport au poids total de l'eau à traiter, de préférence allant de 0,1 % à 5 % en poids, et préférentiellement allant de 0,1 % à 2,5 % en poids par rapport au poids total de l'eau à traiter.

L'invention est illustrée plus en détail dans l'exemple suivant. Les teneurs des ingrédients sont exprimées en pourcentage pondéral.

**EXEMPLES**

**<u>Exemple 1: Détermination de la synergie d'activité antimicrobienne en CMI</u>**

La démonstration d'un effet de synergie d'activité antimicrobienne avec un mélange de 4-(3-éthoxy-4-hydroxyphényl)butan-2-one (appelée substance A) et d'un composé alcool (appelée substance B) a été réalisée par le calcul de l'indice de synergie (ou FIC index), selon la formule suivante :

[Math. 1]

$$\text{FIC Index} = (\text{CMI de A avec B / CMI de A}) + (\text{CMI de B avec A/ CMI de B})$$

avec :

- CMI de A avec B : concentration minimale en produit A dans l'association A + B permettant d'obtenir un effet inhibiteur ;
- CMI de B avec A : concentration minimale en produit B dans l'association A + B permettant d'obtenir l'effet inhibiteur ;
- CMI de A : concentration minimale inhibitrice du produit A seul ;
- CMI de B : concentration minimale inhibitrice du produit B seul.

Cette formule a été décrite pour la première fois dans l'article de F.C. Kull, P.C. Eisman, H.D. Sylwestrowka, and R.L. Mayer, Applied Microbiology 9:538-541, 1961.

Pour chaque composé testé seul, la CMI est considérée comme la première concentration permettant l'obtention d'un pourcentage de croissance microbienne inférieur ou égal à 25 %.

Concernant les associations testées, CMI de A avec B et CMI de B avec A sont les concentrations respectives de A et de B dans les associations permettant l'obtention d'un pourcentage de croissance microbienne inférieur ou égal à 25%.

**<u>Interprétation du FIC</u> Index** :

Lorsque la valeur du FIC index est inférieure ou égale à 1, on considère que l'association des composés testés présente un effet synergique.

La synthèse des résultats obtenus est présentée dans les tableaux suivants.

L'association des composés A et B, et les compositions les contenant ont été testées sur les souches suivantes ou une partie : *Aspergillus niger, Escherichia coli, Staphylococcus aureus, et Candida albicans.*

On a utilisé la souche microbienne *Aspergillus niger* ATCC 6275, et un milieu de culture liquide bouillon Sabouraud additionné de monopalmitate de sorbitane polyoxyéthyléné (20 OE) (Tween 40 de chez Croda) et de Phytagel© BioReagent, à double concentration (soit un mélange de 5 g de Phytagel +0,6 g tween 40 + 60 g bouillon Sabouraud).

On a utilisé la souche microbienne *Staphylococcus aureus* ATCC 6538 et un milieu de culture liquide bouillon nutritif à double concentration.

On a utilisé la souche microbienne *Candida albicans* ATCC 10231 et un milieu de culture liquide bouillon Sabouraud, à double concentration (soit un mélange de 5 g de Phytagel + 0,6 g tween 40 + 60 g bouillon Sabouraud).

On utilise une microplaque 96 puits à une température d'incubation de 32,5 °C.

La durée d'incubation de la microplaque est :

- de 24 à 30 h en aérobiose pour microbienne *Aspergillus niger* ATCC 6275.

- de 18 à 24h en aérobiose pour *Candida albicans* ATCC 10231, *Pseudomonas aeruginosa* ATCC 9027 et *Staphylococcus aureus* ATCC 6538.

## Essais

Pour chaque composé :

A = 4-(3-éthoxy-4-hydroxyphényl)butan-2-one

B = 4-hydroxyacétophénone

On a préparé une solution mère à 10 % (poids/volume) en mélangeant 1 g de composé dans 9 ml de solution aqueuse d'agar à 1 ‰. Des dilutions successives ont été effectuées avec la solution d'agar à 1 ‰.

## Essais des composés A et B seul

50 µL de chacune des solutions filles obtenues contenant le composé A ou B est ajouté dans les puits de la microplaque. On y ajoute également 100 µL de bouillon nutritif liquide de Sabouraud ensemencé à double concentration par la souche *Aspergillus niger* et 50 µL de solution aqueuse d'agar à 1‰.

## Essais des composés A et B en mélange

50 µL de chacune des solutions filles obtenues contenant le composé A et 50 µL de chacune des solutions filles obtenues contenant le composé B sont ajoutées dans les puits de la microplaque. On y ajoute également 100 µL de bouillon nutritif liquide de Sabouraud ensemencé à double concentration par la souche *Aspergillus niger.*

## Témoin de croissance microbienne

On a également réalisé un témoin positif de croissance microbienne. Le témoin positif de croissance microbienne correspond au mélange de 100 µL d'une solution aqueuse d'agar 1‰ avec 100 µL de bouillon nutritif liquide de Sabouraud ensemencé à double concentration par la souche *Aspergillus niger* en l'absence des composés A et B.

## Témoin d'absorbance des composés A et B seul

On a réalisé parallèlement un témoin d'absorbance des composés A et B seul. Ce témoin correspond à 100 µL de bouillon nutritif liquide de Sabouraud stérile à double concentration + 100 µL du composé A ou B à double concentration.
Dans les trois cas (témoin absorbance, témoin de croissance et essai) le volume final présent dans chacun des puits de la microplaque est de 200 µL.
Dans les deux cas (essai et témoin), l'inoculum représente la concentration de la souche *Aspergillus niger* présente dans le volume final des puits (200 µL) et est compris entre 2 et $6.10^5$ UFC/mL en *Aspergillus niger.*
La concentration minimale inhibitrice (CMI) de chaque composé A et B seul et en association a été déterminée de façon connue à l'aide des mesures de densité optique à la longueur d'onde de 620 nm.
Le test tel que décrit ci-dessus (essais, témoins absorbance et témoin de croissance) a de nouveau été réalisé pour tester l'association A + B sur les souches suivantes : *Aspergillus niger, Escherichia coli, Staphylococcus aureus, et Candida albicans.*
On a obtenu les résultats suivants avec le composé B = 4-hydroxy acétophénone :

*Aspergilus niger*

[Tableaux 1]

| Concentrations testées (en % poids) | 0 A | 0,025 A | 0,05 A | 0,1 A |
|---|---|---|---|---|
| 0 B | - | 82 | 43 | 5 |
| 0,0625 B | 86 | 71 | 46 | 3 |
| 0,125 B | 72 | 73 | 20 (FIC 0,75) | 2 |

(suite)

| Concentrations testées (en % poids) | 0 A | 0,025 A | 0,05 A | 0,1 A |
|---|---|---|---|---|
| 0,25 B | 79 | 22 (FIC 0,75) | 12 (FIC 1) | 5 |
| 0,5 B | 6 | 2 (FIC 0,75) | -19 (FIC 1) | 6 |

[Tableaux 2]

| CMI de A seul en % | CMI de B seul en % | CMI de chaque composé en mélange | | FIC Index | Ratio A/B |
|---|---|---|---|---|---|
| | | A % | B % | | |
| 0,1 | 0,5 | 0,05 | 0,25 | 1 | 0,2 |

Les résultats obtenus montrent une amélioration de propriété antimicrobienne, et notamment une synergie de l'activité inhibitrice pour les mélanges suivants :

0,05 % de A et 0,25 % de B soit ratio A/B = 0,2

0,05 % de A et 0,125 % de B soit ratio A/B = 0,4

0,025 % de A et 0,25 % de B soit ratio A/B = 0,1

Les propriété antimicrobiennes ont été également évaluées avec d'autres compositions, telles que celles détaillées ci-dessous dans le tableau 3 dans lesquelles les ingrédients sont donnés en poids (g) pour 100 g de composition. Les propriétés ont été évaluées à 7 jours, 14 jours et 1 mois.

[tableau 3]

| Ingrédients | Composition 1 Invention | Composition 2 Comparative | Composition 3 Comparative |
|---|---|---|---|
| A | 0,7 | 0,7 | - |
| B | 0,2 | - | 0,2 |
| Stéarate de propylène glycol (20 OE) | 0,8 | 0,8 | 0,8 |
| Mélange de mono/distéarate de glycéryle / stéarate de polyéthylène glycol (100 OE) | 2 | 2 | 2 |
| Acide gras (ac. Stéarique majoritaire) d'origine végétale | 3 | 3 | 3 |
| Alcool cétylique | 0,5 | 0,5 | 0,5 |
| Alcool stéarylique | 0,5 | 0,5 | 0,5 |
| Myristate de myristyle | 2 | 2 | 2 |
| Cire d'abeille blanche | 1 | 1 | 1 |
| Beurre de karité | 2 | 2 | 2 |
| Mélange de triglycérides d'acide caprylique et caprique | 3,1 | 3,1 | 3,1 |
| Isostéarate d'isopropyle | 1,2 | 1,2 | 1,2 |
| Cyclohexadiméthylsiloxane | 6 | 6 | 6 |
| Glycérine | 7,0 | 7,0 | 7,0 |
| Copolymère d'acrylamide / acrylamido-2-méthyl propane sulfonate de sodium POLYSORBATE 80/I-C16 | 2,2 | 2,2 | 2,2 |
| Pigment minéral | 0,15 | 0,15 | 0,15 |
| Pigment organique | 0,01 | 0,01 | 0,01 |
| Triétanolamine (agent pH basique) | 0,15 | 0,15 | 0,15 |

(suite)

| Ingrédients | Composition 1 Invention | Composition 2 Comparative | Composition 3 Comparative |
|---|---|---|---|
| Vitamine E : DL-$\alpha$-tocophérol | 0,5 | 0,5 | 0,5 |
| Eau | Qsp 100 | Qsp 100 | Qsp 100 |

Les résultats sur la souche microbienne *Escherichia coli à* 7 jours sont donnés dans le tableau ci-dessous :

[tableau 4]

| Compositions | Après 7 jours nombre de microbes |
|---|---|
| Composition 1 (invention) | *<200* |
| Composition 2 (comparatif) | $2,4.10^4$ |
| Composition 3 (comparatif) | $2,1.10^5$ |

Les résultats sur la souche microbienne *Staphylococcus aureus* à 7 et 14 jours sont donnés dans le tableau ci-dessous :

[Tableau 5]

| Compositions | Après 7 jours nombre de microbes | Après 7 jours nombre de microbes |
|---|---|---|
| Composition 1 (invention) | $3,7.10^5$ | $3,4.10^3$ |
| Composition 2 (comparatif) | $2,4.10^6$ | $9,6.10^5$ |
| Composition 3 (comparatif) | $1,9.10^6$ | $3,0.10^4$ |

Les résultats sur d'autres souches microbiennes à 7, 14 jours puis 1 mois sont donnés dans le tableau ci-dessous :

[Tableau 6]

| Compositions / Souches ciblées | Après 7 jours | Après 14 jours | Après 1 mois |
|---|---|---|---|
| Composition 1 (invention)<br>- *Candida albicans*<br>- *Aspergillus niger* | <br>$8,6.10^3$<br>$6,8.10^5$ | <br>*<200*<br>$2.0.10^3$ | <br>*<200*<br>*<200* |
| Composition 2 (comparatif)<br>- *Candida albicans*<br>- *Aspergillus niger* | <br>$2,0.10^5$<br>$4,1.10^6$ | <br>$8,2.10^4$<br>$5,0.10^6$ | <br>$3,0.10^3$<br>$5,4.10^6$ |
| Composition 3 (comparatif)<br>- *Candida albicans*<br>- *Aspergillus niger* | <br>$3,4.10^5$<br>$3,4.10^6$ | <br>$1.10^5$<br>$3,4.10^6$ | <br>$3,8.10^4$<br>$2,3.10^6$ |

Il apparait des résultats des tableaux ci-dessus que l'association de A et B selon l'invention permet une nette amélioration antimicrobienne et ce après 7 jours, 15 jours, voire un mois pour une large variété de souches microbiennes (*Aspergillus niger, Escherichia coli, Staphylococcus aureus, et Candida albicans).*

**Revendications**

1. Mélange antimicrobien comprenant la i) 4-(3-éthoxy-4-hydroxyphényl)butan-2-one ainsi que ses sels d'acide ou de base, organique ou minéral, ou ses solvates tels que les hydrates avec la ii) 4-hydroxy-acétophénone ainsi que ses sels d'acide ou de base ou ses solvates tels que les hydrates.

2. Mélange selon la revendication précédente dans lequel le ratio pondéral i) / ii) va de 0,05 à 5, de préférence va de 0,08 à 5, préférentiellement va de 0,08 à 0,25, et de 2 à 4 plus préférentiellement va de 0,15 à 0,25 et de 3 à 3,8.

3. Mélange selon la revendication 1 dans lequel le ratio pondéral i) / ii) va de 0,08 à 0,5, de préférence va de 0,08 à 0,3, préférentiellement va de 0,08 à 0,25, plus préférentiellement va de 0,15 à 0,25.

4. Mélange selon la revendication 1 dans lequel le ratio pondéral i) / ii) va de 0,5 à 5, préférentiellement va de 1 à 4 et plus préférentiellement 3 à 3,8.

5. Composition comprenant, la i) 4-(3-éthoxy-4-hydroxyphényl)butan-2-one ainsi que ses sels d'acide ou de base, organique ou minéral, ou ses solvates tels que les hydrates avec la ii) 4-hydroxy-acétophénone ainsi que ses sels d'acide ou de base ou ses solvates tels que les hydrates, préférentiellement dans un ratio pondéral i)/ii) tel que défini dans une des revendications 2 à 4.

6. Composition selon la revendication précédente qui comprend un ou plusieurs tensioactifs non ioniques, anioniques, cationiques, zwitterioniques ou amphotères, particulièrement choisis parmi les tensioactifs non ioniques, anioniques et leur mélange.

7. Composition selon la revendication 5 ou 6 qui comprend un ou plusieurs tensioactifs non ioniques choisis parmi : les alcools gras (poly)éthoxylés ; les alcools gras glycérolés ; les alkylpolyglycosides. de préférence les mono et diesters d'acides gras du sorbitan oxyéthylénés ayant de 2 à 200 moles OE, en particulier de 10 à 100 OE, tel que le stéarate de propylène glycol ayant de 10 à 30 OE tel que 20 OE et les mono distéarate de glycéryle / stéarate de polyéthylène glycol (100 OE), ou leur mélange.

8. Composition selon une quelconque des revendications 5 à 7 qui comprend un ou plusieurs tensioactifs anioniques choisis parmi : les acides alkyl carboxyliques, les alkyl sulfates, les alkyl éther sulfates, les alkylamidoéthersulfates, les alkylarylpolyéthersulfates, les monoglycéride-sulfates, les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les sels de diesters d'alkyle et d'acides polyglycoside-polycarboxyliques, les acyllactylates, les sels d'acides D-galactoside-uroniques, les sels d'acides alkyl éther-carboxyliques, les sels d'acides alkylaryl éther-carboxyliques, les sels d'acides alkyl amidoéther-carboxyliques, et les formes non salifiées correspondantes de tous ces composés, les groupes alkyle et acyle de tous ces composés comportant de 8 à 30 atomes de carbone, de préférence de 10 à 22 atomes de carbone et le groupe aryle désignant un groupe phényle qui peuvent être oxyéthylénés, de préférence le ou les tensioactifs anioniques sont choisis parmi les acides alkyl($C_6$-$C_{24}$)carboxyliques, en particulier acides alkyl($C_{10}$-$C_{20}$)carboxyliques, de préférence d'origine naturelle en particulier végétale tel que acide stéarique, pouvant être sous forme de sels de métaux alcalins, d'ammonium, d'aminoalcools, et de métaux alcalino-terreux, ou un mélange de ces composés.

9. Composition selon une quelconque des revendications 6 à 8 dans laquelle la quantité de tensioactifs va de préférence de 0,5 % à 30 % en poids, en particulier de 1 % à 20 % en poids, et plus particulièrement de 2 à 10 % en poids, plus préférentiellement entre 4 et 6 % rapportée au poids total de la composition de l'invention.

10. Composition selon une quelconque des revendications 5 à 9 qui comprend un ou plusieurs corps gras de préférence choisi parmi :

a) les beurres de préférence le beurre de karité,
b) les cires de préférence cires d'abeilles,
c) les alcools gras non liquides, particulièrement choisis parmi les alcools saturés ou insaturés, linéaires ou ramifiés, comportant de 8 à 30 atomes de carbone, préférentiellement en $C_{10}$-$C_{22}$, plus préférentiellement en $C_{14}$-$C_{20}$, mieux en $C_{16}$-$C_{18}$ tel que l'alcool cétylique et l'alcool stéarylique et leur mélange,
d) les esters d'acide gras et/ou d'alcools gras non liquides, notamment les esters solides issus d'acides gras en $C_9$-$C_{26}$ et d'alcools gras en $C_9$-$C_{26}$, en particulier les myristates d'alkyles tels que le myristate de cétyle, de mirystyle, de stéaryle ; le stéarate d'hexyle, plus particulièrement le myrystate de myristyle ;
e) les esters de monoalcools, dont un au moins de l'alcool ou de l'acide sont issus desdits esters est ramifié tels que les palmitates d'éthyle et d'isopropyle, les myristates d'alkyles tels que le myristate d'isopropyle, d'éthyle, le stéarate d'isocétyle, l'isononanoate d'éthyl-2-hexyle, le néopentanoate d'isodécyle, le néopentanoate d'isostéaryle, et les (iso)stéarates d'alkyles en $C_{10}$-$C_{22}$, de préférence en $C_{12}$-$C_{20}$ tels que l'isostéarate d'isopropyle ;
f) les polydialkylsiloxanes cycliques comportant de 3 à 7, de préférence de 4 à 5 atomes de silicium tel que l'octaméthylcyclotétrasiloxane, le décaméthylcyclopentasiloxane, le dodécaméthylcyclopentasiloxane, ou le cyclohexadiméthylsiloxane ainsi que leurs mélanges, de préférence cyclohexadiméthylsiloxane ;
g) les huiles d'origine végétale ou triglycérides synthétiques, telles que les triglycérides liquides d'acides gras comportant de 6 à 30 atomes de carbone comme les triglycérides des acides heptanoïque ou octanoïque ou encore, les huiles de tournesol, de maïs, de soja, de courge, de pépins de raisin, de sésame, de noisette, d'abricot, de macadamia, d'arara, de tournesol, de ricin, d'avocat, les triglycérides des acides caprylique / caprique, l'huile de jojoba, l'huile de beurre de karité.

**11.** Composition selon une quelconque des revendications 5 à 10 qui comprend un ou plusieurs corps gras dans une quantité variant de 1 à 40 % en poids, de préférence en une quantité variant de 5 à 30 % en poids, et encore plus préférentiellement dans une quantité variant de 10 à 20 % en poids par rapport au poids total de la composition.

**12.** Composition selon une quelconque des revendications 5 à 11 qui comprend un ou plusieurs polymères organiques épaississants en particulier d'origine naturelle ou synthétique, anioniques, cationiques, amphotères ou non ioniques, associatifs ou non associatif, de préférence non associatifs.

**13.** Composition selon une quelconque des revendications 5 à 12 qui comprend un ou plusieurs polymères organiques épaississants issus de la (co)polymérisation :

- de monomère acrylate $CH_2$=C(R')-COOR''' (VIa) et/ou
- de monomère acrylamide $CH_2$=C(R')-CO-N(R")-L-$Y^-$ $M^+$ (VIb) Formules (Via) et (VIb) dans lesquelles :

  * R', et R", identiques ou différents, représentant un atome d'hydrogène ou un groupe ($C_1$-$C_6$)alkyle tel que méthyle, de préférence hydrogène,
  * R''' représente un métal alcalin, un métal alcalino-terreux, un atome d'hydrogène ou un groupe ($C_1$-$C_6$) alkyle éventuellement substitué notamment par un ou plusieurs groupes hydroxy, carboxy ou amino ;
  * L représentant un groupe hydrocarboné divalent, cyclique ou acylique, saturé ou insaturé, linéaire ou ramifié, éventuellement interrompu et/ou substitué par un ou plusieurs hétéroatomes tels que O, N et comprenant de 1 à 20 atomes de carbones, de préférence de 1 à 6 atomes de carbones, de préférence L représente le groupe divalent -$[C(R')(R")]_p$- avec p représentant un entier compris entre 1 et 4, de préférence 2 et 3 tel que 2, R' et R" étant tels que définis précédemment, plus particulièrement L représente -C(R')(R")-$CH_2$- ou -$CH_2$-C(R')(R")- avec R' et R" tels que définis précédemment, de préférence R' et R" représentent un groupe ($C_1$-$C_4$)alkyle tel que méthyle ;
  * $Y^-$ représente un groupe anionique tel que carboxylate, phosphate, phosphonate, sulfonate, ou sulfate de préférence -$S(O)_2$-$O^-$, et $M^+$ étant un contre ion cationique de préférence métal alcalin tel que sodium, ledit copolymère pouvant se trouver en émulsion directe ou inverse, de préférence inverse ;

plus préférentiellement le ou les polymères organiques épaississants de l'invention sont issus de la copolymérisation de monomère acrylate $CH_2$=C(R')-COOH (VIa) et de monomère acrylamide $CH_2$=C(R')-CO-N(R")-L-$Y^-$ $M^+$ (VIb) tels que définis précédemment ; de préférence les polymères organiques épaississants sont choisis parmi les copolymères d'acide acrylique ou méthacryliques réticulés ou non, homopolymères réticulés ou non d'acide 2-acrylamido-2-méthyl-propane sulfonique et leurs copolymères réticulés ou non d'acrylamide, les homopolymères d'acrylate d'ammonium ou les copolymères d'acrylate d'ammonium et d'acrylamide seuls ou en mélanges.

**14.** Composition selon une quelconque des revendications 5 à 13 qui comprend un ou plusieurs polymères organiques épaississants à motifs sucres en particulier issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose, plus particulièrement de galactose et d'anhydrogalactose, de préférence le ou les polymères organiques épaississants à motifs sucres sont choisis parmi l'agar.

**15.** Composition selon une quelconque des revendications 5 à 14 qui comprend un ou plusieurs polymères organiques épaississants dans une teneur allant de 0,01 à 10 % en poids plus préférentiellement de 0,1 à 5 % en poids par rapport au poids total de la composition.

**16.** Composition selon une quelconque des revendications 5 à 15 qui comprend un ou plusieurs corps gras tels que définis dans les revendications dans les revendications 10 ou 11, et un ou plusieurs tensioactifs tels que définis dans

une quelconque des revendications 6 à 9 dont le ratio pondéral corps gras / tensioactif(s) est compris inclusivement entre 5 et 20, de préférence entre 8 et 15, encore plus préférentiellement entre 10 et 13 tel que 11,8.

**17.** Composition selon une quelconque des revendications 5 à 16 qui comprend un ou plusieurs corps gras tels que définis dans les revendications dans les revendications 10 ou 11, et un ou plusieurs tensioactifs tels que définis dans une quelconque des revendications 6 à 9, et un ou plusieurs polymères organiques épaississants tels que définis dans une quelconque des revendications 12 à 15 dont le ratio pondéral en corps gras / somme de tensioactif(s) et polymère(s) [tensioactif(s) + polymère] est compris inclusivement entre 0,8 et 10, particulièrement entre 1 et 5, plus particulièrement entre 1,5 et 2,5, tel que 1,9.

**18.** Composition selon une quelconque des revendications 5 à 17 qui comprend une phase aqueuse et une phase organique ou huileuse, de préférence la composition de l'invention est une émulsion directe de type huile dans eau (H/E).

**19.** Composition selon une quelconque des revendications 5, 10 à 15 qui est aqueuse et ne comprend pas de tensioactifs.

**20.** Composition selon une quelconque des revendications 5 à 19 qui comprend un ou plusieurs solvants organiques en particulier choisis parmi a) les alcanols en $C_2$-$C_6$, tels que l'éthanol et l'isopropanol ; b) les polyols miscibles à l'eau à la température ambiante (25 °C) notamment choisi parmi les polyols ayant notamment de 2 à 10 atomes de carbones, de préférence ayant de 2 à 6 atomes de carbone, tels que la glycérine, le propylène glycol, le 1,3-propanediol, le butylène glycol, le pentylène glycol, l'hexylène glycol, le dipropylène glycol, le diéthylène glycol, la diglycérine ; c) les éthers de polyols comme le 2-butoxyéthanol, le monométhyléther de propylèneglycol, le monoéthyléther et le monométhyléther du diéthylèneglycol, ainsi que d) les alcools aromatiques comme l'alcool benzylique ou le phénoxyéthanol, et leurs mélanges, de préférence la composition comprend en un ou plusieurs polyols notamment choisi parmi les polyols ayant notamment de 2 à 10 atomes de carbones, de préférence ayant de 2 à 6 atomes de carbone, tels que la glycérine.

**21.** Composition selon une quelconque des revendications 5 à 20 qui comprend un ou plusieurs solvants organiques présents dans des quantités de comprise inclusivement entre 0,1 et 40 % en poids environ par rapport au poids total de la composition, et plus préférentiellement entre 1 et 20 % en poids environ et encore plus particulièrement compris inclusivement entre 5% et 10 % en poids par rapport au poids total de la composition.

**22.** Procédé de traitement cosmétique non thérapeutique de soin et/ou de maquillage et/ou de nettoyage des matières kératiniques comprenant l'application sur lesdites matières kératiniques d'une composition selon l'une quelconque des revendications 5 à 21.

**23.** Procédé de conservation d'une composition comprenant un milieu physiologiquement acceptable, en particulier une composition cosmétique ou dermatologique, **caractérisé en ce qu'**il consiste à incorporer à ladite composition un mélange antimicrobien tel que défini à l'une des revendications 1 à 4, ou une composition telle que définie dans une quelconque des revendications 5 à 21.

**Patentansprüche**

**1.** Antimikrobielle Mischung, umfassend i) 4-(3-Ethoxy-4-hydroxyphenyl)butan-2-on sowie organische oder anorganische Säure- oder Basensalze oder Solvate davon wie Hydrate mit ii) 4-Hydroxyacetophenon sowie Säure- oder Basensalze davon oder Solvate davon wie Hydrate.

**2.** Mischung nach dem vorhergehenden Anspruch, wobei das Gewichtsverhältnis i)/ii) im Bereich von 0,05 bis 5, vorzugsweise im Bereich von 0,08 bis 5, bevorzugt im Bereich von 0,08 bis 0,25 und von 2 bis 4, weiter bevorzugt im Bereich von 0,15 bis 0,25 und 3 bis 3,8, liegt.

**3.** Mischung nach dem Anspruch 1, wobei das Gewichtsverhältnis i)/ii) im Bereich von 0,08 bis 0,5, vorzugsweise im Bereich von 0,08 bis 0,3, bevorzugt im Bereich von 0,08 bis 0,25, weiter bevorzugt im Bereich von 0,15 bis 0,25, liegt.

**4.** Mischung nach dem Anspruch 1, wobei das Gewichtsverhältnis i)/ii) im Bereich von 0,5 bis 5, bevorzugt im Bereich von 1 bis 4 und weiter bevorzugt 3 bis 3,8 liegt.

5. Zusammensetzung, umfassend i) 4-(3-Ethoxy-4-hydroxyphenyl)butan-2-on sowie organische oder anorganische Säure- oder Basensalze oder Solvate davon wie Hydrate mit ii) 4-Hydroxyacetophenon sowie Säure- oder Basensalze davon oder Solvate davon wie Hydrate, bevorzugt in einem wie in einem der Ansprüche 2 bis 4 definierten Gewichtsverhältnis i)/ii).

6. Zusammensetzung nach dem vorhergehenden Anspruch, die ein oder mehrere nichtionische, anionische, kationische, zwitterionische oder amphotere Tenside, insbesondere ausgewählt aus nichtionischen und anionischen Tensiden, und eine Mischung davon umfasst.

7. Zusammensetzung nach Anspruch 5 oder 6, die ein oder mehrere nichtionische Tenside umfasst, ausgewählt aus: (poly)ethoxylierten Fettalkoholen; glycerinierten Fettalkoholen; Alkylpolyglykosiden, vorzugsweise oxyethylenierten Sorbitanfettsäuremonoestern und - diestern mit 2 bis 200 mol EO, insbesondere 10 bis 100 EO, wie Propylenglykolstearat mit 10 bis 30 EO, wie 20 EO, und Glycerylmono-/distearat/Polyethylenglykolstearat (100 EO) oder einer Mischung davon.

8. Zusammensetzung nach einem der Ansprüche 5 bis 7, die ein oder mehrere anionische Tenside umfasst, ausgewählt aus: Alkylcarbonsäuren, Alkylsulfaten, Alkylethersulfaten, Alkylamidoethersulfaten, Alkylarylpolyethersulfaten, Monoglyceridsulfaten, Alkylsulfonaten, Alkylamidsulfonaten, Alkylarylsulfonaten, alpha-Olefinsulfonaten, Paraffinsulfonaten, Alkylsulfosuccinaten, Alkylethersulfosuccinaten, Alkylamidsulfosuccinaten, Alkylsulfoacetaten, Acylsarkosinaten, Acylglutamaten, Alkylsulfosuccinamaten, Acylisethionaten und N-Acyltauraten, Salzen von Alkylmonoestern von Polyglykosidpolycarbonsäuren, Salzen von Alkyldiestern von Polyglykosidpolycarbonsäuren, Acyllactylaten, D-Galactosiduronsäuresalzen, Alkylethercarbonsäuresalzen, Alkylarylethercarbonsäuresalzen, Alkylamidoethercarbonsäuresalzen und den entsprechenden unversalzten Formen all dieser Verbindungen, wobei die Alkyl- und Acylgruppen all dieser Verbindungen 8 bis 30 Kohlenstoffatome und vorzugsweise 10 bis 22 Kohlenstoffatome umfassen und die Arylgruppe eine Phenylgruppe bezeichnet, die oxyethyleniert sein kann; vorzugsweise die anionischen Tenside ausgewählt sind aus ($C_6$-$C_{24}$)-Alkylcarbonsäuren, insbesondere ($C_{10}$-$C_{20}$)-Alkylcarbonsäuren, vorzugsweise natürlichen Ursprungs, insbesondere pflanzlichen Ursprungs, wie Stearinsäure, die in Form von Alkalimetall-, Ammonium-, Aminoalkohol- und Erdalkalimetallsalzen vorliegen können, oder einer Mischung dieser Verbindungen.

9. Zusammensetzung nach einem der Ansprüche 6 bis 8, wobei die Menge an Tensiden vorzugsweise im Bereich von 0,5 bis 30 Gew.-%, insbesondere von 1 bis 20 Gew.-% und spezieller von 2 bis 10 Gew.-%, weiter bevorzugt zwischen 4 und 6 %, bezogen auf das Gesamtgewicht der erfindungsgemäßen Zusammensetzung, liegt.

10. Zusammensetzung nach einem der Ansprüche 5 bis 9, die ein oder mehrere Fettsubstanzen umfasst, vorzugsweise ausgewählt aus:

a) Butter, vorzugsweise Sheabutter,
b) Wachsen, vorzugsweise Bienenwachs,
c) nichtflüssigen Fettalkoholen, insbesondere ausgewählt aus gesättigten oder ungesättigten, linearen oder verzweigten Alkoholen mit 8 bis 30 Kohlenstoffatomen, bevorzugt $C_{10}$-$C_{22}$, weiter bevorzugt $C_{14}$-$C_{20}$, noch besser $C_{16}$-$C_{18}$ wie Cetylalkohol und Stearylalkohol, und einer Mischung davon,
d) nichtflüssigen Fettsäure- und/oder Fettalkoholestern, insbesondere festen Estern, die sich von $C_9$-$C_{26}$-Fettsäuren und $C_9$-$C_{26}$-Fettalkoholen ableiten, insbesondere Alkylmyristaten wie Cetyl-, Myristyl- oder Stearylmyristat; Hexylstearat, spezieller Myristylmyristat;
e) Estern von Monoalkoholen, wobei der Alkohol und/oder die Säure, von dem bzw. der sich diese Ester ableiten, verzweigt ist bzw. sind, wie Ethylpalmitat, Isopropylpalmitat, Alkylmyristaten wie Isopropyl- oder Ethylmyristat, Isocetylstearat, 2-Ethylhexylisononanoat, Isodecylneopentanoat, Isostearylneopentanoat und $C_{10}$-$C_{22}$- und vorzugsweise $C_{12}$-$C_{20}$-Alkyl(iso) stearate wie Isopropylisostearat;
f) cyclischen Polydialkylsiloxanen mit 3 bis 7 und vorzugsweise 4 bis 5 Siliciumatomen wie Octamethylcyclotetrasiloxan, Decamethylcyclopentasiloxan, Dodecamethylcyclopentasiloxan oder Cyclohexadimethylsiloxan sowie Mischungen davon, vorzugsweise Cyclohexadimethylsiloxan;
g) Ölen pflanzlichen Ursprungs oder synthetischen Triglyceriden, wie flüssigen Triglyceriden von Fettsäuren mit 6 bis 30 Kohlenstoffatomen wie Heptansäure- oder Octansäuretriglyceriden, oder alternativ dazu Sonnenblumenöl, Maisöl, Sojaöl, Marköl, Traubenkernöl, Sesamöl, Haselnussöl, Aprikosenöl, Macadamiaöl, Araraöl, Sonnenblumenöl, Rizinusöl, Avocadoöl, Capryl-/Caprinsäuretriglyceriden, Jojobaöl, Sheabutteröl.

11. Zusammensetzung nach einem der Ansprüche 5 bis 10, die eine oder mehrere Fettsubstanzen in einer Menge im

Bereich von 1 bis 40 Gew.-%, vorzugsweise in einer Menge im Bereich von 5 bis 30 Gew.-%, und noch weiter bevorzugt in einer Menge im Bereich von 10 bis 20 Gew.-%, bezogen auf das Gesamtgewicht der Zusammensetzung, umfasst.

**12.** Zusammensetzung nach einem der Ansprüche 5 bis 11, die ein oder mehrere verdickende organische Polymere, insbesondere natürlichen oder synthetischen Ursprungs, die assoziativ oder nichtassoziativ, vorzugsweise nichtassoziativ, anionisch, kationisch, amphoter oder nichtionisch sind, umfasst.

**13.** Zusammensetzung nach einem der Ansprüche 5 bis 12, die ein oder mehrere Polymere umfasst, die aus der (Co) polymerisation von Folgendem abgeleitet sind:

- Acrylatmonomer $CH_2$=C(R')-COOR''' (VIa) und/oder
- Acrylamidmonomer $CH_2$=C(R')-CO-N(R'')-L-Y-$M^+$ (VIb) wobei in den Formeln (VIa) und (VIb):

  * R' und R'', die gleich oder verschieden sind, für ein Wasserstoffatom oder eine ($C_1$-$C_6$)-Alkylgruppe wie Methyl, vorzugsweise Wasserstoff, stehen,
  * R''' für ein Alkalimetall, ein Erdalkalimetall, ein Wasserstoffatom oder eine ($C_1$-$C_6$)-Alkylgruppe, die gegebenenfalls substituiert ist, insbesondere durch eine oder mehrere Hydroxy-, Carboxy- oder Aminogruppen, steht;
  * L für eine lineare oder verzweigte, gesättigte oder ungesättigte, cyclische oder acyclische zweiwertige Kohlenwasserstoffgruppe, die gegebenenfalls durch ein oder mehrere Heteroatome wie O, N unterbrochen und/oder substituiert ist und 1 bis 20 Kohlenstoffatome, vorzugweise 1 bis 6 Kohlenstoffatome, umfasst, steht, vorzugsweise L für die zweiwertige Gruppe -$[C(R')(R'')]_p$- steht, wobei p für eine ganze Zahl zwischen 1 und 4, vorzugsweise 2 und 3, wie 2, steht, R' und R" wie oben definiert sind, spezieller L für -C(R')(R")-$CH_2$- oder - $CH_2$-C(R')(R")- steht, wobei R' und R" wie oben definiert sind, vorzugsweise R' und R" für eine ($C_1$-$C_4$)-Alkylgruppe wie Methyl stehen;
  * $Y^-$ für eine anionische Gruppe wie Carboxylat, Phosphat, Phosphonat, Sulfonat oder Sulfat, vorzugsweise -$S(O)_2$-$O^-$, steht und $M^+$ ein kationisches Gegenion, vorzugsweise ein Alkalimetall wie Natrium, ist, wobei das Copolymer in direkter oder inverser, vorzugsweise inverser, Emulsion vorliegen kann;

weiter bevorzugt das bzw. die verdickenden organischen Polymere der Erfindung abgeleitet sind aus der Copolymerisation von Acrylatmonomer $CH_2$=C(R')-COOH (VIa) und Acrylamidmonomer $CH_2$=C(R')-CO-N(R'')-L-$Y^-$ $M^+$ (VIb) gemäß obiger Definition; vorzugsweise die verdickenden organischen Polymere aus vernetzten oder unvernetzten Acrylsäure- oder Methacrylsäure-Copolymeren, vernetzten oder unvernetzten 2-Acrylamido-2-methylpropansulfonsäure-Homopolymeren und vernetzten oder unvernetzten Acrylamid-Copolymeren, Ammoniumacrylat-Homopolymeren oder Copolymeren von Ammoniumacrylat und Acrylamid alleine oder als Mischungen ausgewählt sind.

**14.** Zusammensetzung nach einem der Ansprüche 5 bis 13, die ein oder mehrere verdickende organische Polymere mit Zuckereinheiten, insbesondere abgeleitet von den folgenden Zuckern, umfasst: Glucose; Galactose; Arabinose; Rhamnose; Mannose; Xylose; Fucose; Anhydrogalactose; Galacturonsäure; Glucuronsäure; Mannuronsäure; Galactosesulfat; Anhydrogalactosesulfat und Fructose, spezieller Galactose und Anhydrogalactose; vorzugsweise das bzw. die verdickenden organischen Polymere mit Zuckereinheiten aus Agar ausgewählt ist bzw. sind.

**15.** Zusammensetzung nach einem der Ansprüche 5 bis 14, die ein oder mehrere verdickende organische Polymere in einem Gehalt im Bereich von 0,01 bis 10 Gew.-%, weiter bevorzugt von 0,1 bis 5 Gew.-%, bezogen auf das Gesamtgewicht der Zusammensetzung, umfasst.

**16.** Zusammensetzung nach einem der Ansprüche 5 bis 15, die eine oder mehrere Fettsubstanzen gemäß den Ansprüchen 10 oder 11 und ein oder mehrere Tenside gemäß einem der Ansprüche 6 bis 9 umfasst, wobei das Gewichtsverhältnis Fettsubstanz/Tensid(e) zwischen 5 und 20 inklusive, vorzugsweise zwischen 8 und 15, noch weiter bevorzugt zwischen 10 und 13, wie 11,8, liegt.

**17.** Zusammensetzung nach einem der Ansprüche 5 bis 16, die eine oder mehrere Fettsubstanzen gemäß den Ansprüchen 10 oder 11 und ein oder mehrere Tenside gemäß einem der Ansprüche 6 bis 9 und ein oder mehrere verdickende organische Polymere gemäß einem der Ansprüche 12 bis 15 umfasst, wobei das Gewichtsverhältnis Fettsubstanz/Summe von Tensid(en) und Polymer(en) [Tensid(e) + Polymer] zwischen 0,8 und 10 inklusive, insbesondere zwischen 1 und 5, spezieller zwischen 1,5 und 2,5, wie 1,9, liegt.

**18.** Zusammensetzung nach einem der Ansprüche 5 bis 17, die eine wässrige Phase und eine organische oder ölige Phase umfasst; vorzugsweise die Zusammensetzung der Erfindung eine direkte Emulsion vom Öl-in-Wasser(O/W)-Typ ist.

**19.** Zusammensetzung nach einem der Ansprüche 5, 10 bis 15, die wässrig ist und keine Tenside enthält.

**20.** Zusammensetzung nach einem der Ansprüche 5 bis 19, die ein oder mehrere organische Lösungsmittel umfasst, insbesondere ausgewählt aus a) $C_2$-$C_6$-Alkanolen, wie Ethanol und Isopropanol; b) Polyolen, die bei Raumtemperatur (25 °C) wassermischbar sind, insbesondere ausgewählt aus Polyolen, die insbesondere 2 bis 10 Kohlenstoffatome, vorzugsweise 2 bis 6 Kohlenstoffatome, aufweisen, wie Glycerin, Propylenglykol, 1,3-Propandiol, Butylenglykol, Pentylenglykol, Hexylenglykol, Dipropylenglykol, Diethylenglykol, Diglycerin; c) Polyolethern, wie 2-Butoxyethanol, Propylenglykolmonomethylether, Diethylenglykolmonoethylether und Diethylenglykolmonomethylether, sowie d) aromatischen Alkoholen, wie Benzylalkohol oder Phenoxyethanol, und Mischungen davon, wobei die Zusammensetzung vorzugsweise ein oder mehrere Polyole umfasst, die insbesondere aus Polyolen, die insbesondere 2 bis 10 Kohlenstoffatome und vorzugsweise 2 bis 6 Kohlenstoffatome aufweisen, wie Glycerin, ausgewählt sind.

**21.** Zusammensetzung nach einem der Ansprüche 5 bis 20, die ein oder mehrere organische Lösungsmittel in Mengen zwischen etwa 0,1 und 40 Gew.-% inklusive, bezogen auf das Gesamtgewicht der Zusammensetzung, und weiter bevorzugt zwischen etwa 1 und 20 Gew.-% und noch spezieller zwischen 5 und 10 % Gew.-% bezogen auf das Gesamtgewicht der Zusammensetzung, umfasst.

**22.** Nichttherapeutisches kosmetisches Behandlungsverfahren zum Pflegen und/oder Schminken und/oder Reinigen von Keratinmaterialien, umfassend das Aufbringen einer Zusammensetzung nach einem der Ansprüche 5 bis 21 auf die Keratinmaterialien.

**23.** Verfahren zum Konservieren einer Zusammensetzung, die ein physiologisch unbedenkliches Medium umfasst, insbesondere einer kosmetischen oder dermatologischen Zusammensetzung, **dadurch gekennzeichnet, dass** es aus dem Einarbeiten einer antimikrobiellen Mischung gemäß einem der Ansprüche 1 bis 4 oder einer Zusammensetzung gemäß einem der Ansprüche 5 bis 21 in die Zusammensetzung besteht.

## Claims

**1.** Antimicrobial mixture comprising i) 4-(3-ethoxy-4-hydroxyphenyl)butan-2-one and also the organic or mineral acid or base salts thereof, or the solvates thereof such as hydrates, with ii) 4-hydroxyacetophenone and also the acid or base salts thereof or the solvates thereof such as hydrates.

**2.** Mixture according to the preceding claim, wherein the weight ratio i)/ii) ranges from 0.05 to 5, preferably ranges from 0.08 to 5, preferentially ranges from 0.08 to 0.25, and from 2 to 4, more preferentially ranges from 0.15 to 0.25 and from 3 to 3.8.

**3.** Mixture according to Claim 1, wherein the weight ratio i)/ii) ranges from 0.08 to 0.5, preferably ranges from 0.08 to 0.3, preferentially ranges from 0.08 to 0.25, more preferentially ranges from 0.15 to 0.25.

**4.** Mixture according to Claim 1, wherein the weight ratio i)/ii) ranges from 0.5 to 5, preferentially range from 1 to 4 and more preferentially 3 to 3.8.

**5.** Composition comprising i) 4-(3-ethoxy-4-hydroxyphenyl)butan-2-one and also the organic or mineral acid or base salts thereof, or the solvates thereof such as hydrates, with ii) 4-hydroxyacetophenone and also the acid or base salts thereof or the solvates thereof such as hydrates, preferentially in a weight ratio i)/ii) as defined in one of Claims 2 to 4.

**6.** Composition according to the preceding claim, which comprises one or more nonionic, anionic, cationic, zwitterionic or amphoteric surfactants, particularly chosen from nonionic and anionic surfactants, and a mixture thereof.

**7.** Composition according to Claim 5 or 6, which comprises one or more nonionic surfactants chosen from: (poly) ethoxylated fatty alcohols; glycerolated fatty alcohols; alkylpolyglycosides, preferably oxyethylenated fatty acid mono- and diesters of sorbitan having from 2 to 200 mol of EO, in particular from 10 to 100 EO, such as propylene

glycol stearate having from 10 to 30 EO, such as 20 EO, and glyceryl monostearate/distearate / polyethylene glycol stearate (100 EO), or a mixture thereof.

**8.** Composition according to any one of Claims 5 to 7, which comprises one or more anionic surfactants chosen from: alkyl carboxylic acids, alkyl sulfates, alkyl ether sulfates, alkylamido ether sulfates, alkylaryl polyether sulfates, monoglyceride sulfates, alkylsulfonates, alkylamidesulfonates, alkylarylsulfonates, $\alpha$-olefinsulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-acyltaurates, salts of alkyl monoesters of polyglycoside-polycarboxylic acids, salts of alkyl diesters of polyglycoside-polycarboxylic acids, acyl lactylates, D-galactoside-uronic acid salts, alkyl ether carboxylic acid salts, alkylaryl ether carboxylic acid salts, alkylamido ether carboxylic acid salts, and the corresponding non-salified forms of all these compounds, the alkyl and acyl groups of all these compounds comprising from 8 to 30 carbon atoms and preferably from 10 to 22 carbon atoms and the aryl group denoting a phenyl group which can be oxyethylenated; preferably the anionic surfactant(s) are chosen from ($C_6$-$C_{24}$) alkyl carboxylic acids, in particular ($C_{10}$-$C_{20}$)alkyl carboxylic acids, preferably of natural origin, in particular of plant origin, such as stearic acid, which may be in the form of alkali metal, ammonium, amino alcohol and alkaline-earth metal salts, or a mixture of these compounds.

**9.** Composition according to any one of Claims 6 to 8, wherein the amount of surfactants preferably ranges from 0.5% to 30% by weight, in particular from 1% to 20% by weight and more particularly from 2% to 10% by weight, more preferentially between 4% and 6%, relative to the total weight of the composition of the invention.

**10.** Composition according to any one of Claims 5 to 9, which comprises one or more fatty substances preferably chosen from:

a) butters, preferably shea butter;
b) waxes, preferably beeswaxes;
c) non-liquid fatty alcohols, particularly chosen from saturated or unsaturated, linear or branched alcohols comprising from 8 to 30 carbon atoms, which are preferentially $C_{10}$-$C_{22}$, more preferentially $C_{14}$-$C_{20}$, better still $C_{16}$-$C_{18}$, such as cetyl alcohol and stearyl alcohol and the mixture thereof;
d) non-liquid fatty acid and/or fatty alcohol esters, in particular solid esters derived from $C_9$-$C_{26}$ fatty acids and from $C_9$-$C_{26}$ fatty alcohols, in particular alkyl myristates such as cetyl, mirystyl or stearyl myristate; hexyl stearate, more particularly myristyl myrystate;
e) esters of monoalcohols, at least one of the alcohol or of the acid of which are derived from said esters is branched, such as ethyl palmitate, isopropyl palmitate, alkyl myristates, such as isopropyl myristate or ethyl myristate, isocetyl stearate, 2-ethylhexyl isononanoate, isodecyl neopentanoate, isostearyl neopentanoate and $C_{10}$-$C_{22}$, preferably $C_{12}$-$C_{20}$, alkyl (iso)stearates, such as isopropyl isostearate;
f) cyclic polydialkylsiloxanes comprising from 3 to 7, preferably from 4 to 5 silicon atoms, such as octamethylcyclotetrasiloxane, decamethylcyclopentasiloxane, dodecamethylcyclopentasiloxane, or cyclohexadimethylsiloxane and also mixtures thereof, preferably cyclohexadimethylsiloxane;
g) oils of plant origin or synthetic triglycerides, such as liquid triglycerides of fatty acids containing from 6 to 30 carbon atoms, for instance heptanoic or octanoic acid triglycerides, or alternatively sunflower oil, corn oil, soybean oil, marrow oil, grapeseed oil, sesame seed oil, hazelnut oil, apricot oil, macadamia oil, arara oil, sunflower oil, castor oil, avocado oil, caprylic/capric acid triglycerides, jojoba oil, shea butter oil.

**11.** Composition according to any one of Claims 5 to 10, which comprises one or more fatty substances in an amount ranging from 1% to 40% by weight, preferably in an amount ranging from 5% to 30% by weight and even more preferentially in an amount ranging from 10% to 20% by weight, relative to the total weight of the composition.

**12.** Composition according to any one of Claims 5 to 11, which comprises one or more thickening organic polymers, in particular of natural or synthetic origin, which are associative or non-associative, preferably non-associative, anionic, cationic, amphoteric or nonionic polymers.

**13.** Composition according to any one of Claims 5 to 12, which comprises one or more thickening organic polymers resulting from the (co)polymerization:

- of acrylate monomer $CH_2$=C(R')-COOR''' (VIa) and/or
- of acrylamide monomer $CH_2$=C(R')-CO-N(R")-L-$Y^-$ $M^+$ (VIb) Formulae (VIa) and (VIb) wherein:

* R’ and R’’, which may be identical or different, represent a hydrogen atom or a ($C_1$-$C_6$)alkyl group such as methyl, preferably hydrogen,
* R’’’ represents an alkali metal, an alkaline-earth metal, a hydrogen atom or a ($C_1$-$C_6$)alkyl group optionally substituted in particular by one or more hydroxyl, carboxy or amino groups;
* L representing a linear or branched, saturated or unsaturated, cyclic or acyclic, divalent hydrocarbon group optionally interrupted and/or substituted by one or more heteroatoms such as O or N and comprising from 1 to 20 carbon atoms, preferably from 1 to 6 carbon atoms, preferably L represents the divalent group - $[C(R')(R'')]_p$- with p representing an integer between 1 and 4, preferably 2 and 3, such as 2, R’ and R’’ being as defined above, more particularly L represents - C(R’)(R")-$CH_2$- or -$CH_2$-C(R’)(R")- with R’ and R" as defined above, preferably R’ and R’’ represent a ($C_1$-$C_4$)alkyl group such as methyl;
* $Y^-$ represents an anionic group such as carboxylate, phosphate, phosphonate, sulfonate or sulfate, preferably

- $S(O)_2$-$O^-$, and $M^+$ being a cationic counterion, preferably an alkali metal such as sodium, said copolymer possibly being in a direct or inverse emulsion, preferably an inverse emulsion;

more preferentially, the thickening organic polymer(s) of the invention result from the copolymerization of an acrylate monomer $CH_2$=C(R’)-COOH (VIa) and an acrylamide monomer $CH_2$=C(R’)-CO-N(R")-L-$Y^-$ $M^+$ (VIb) as defined above; preferably, the thickening organic polymers are chosen from crosslinked or non-crosslinked acrylic acid or methacrylic acid copolymers, crosslinked or non-crosslinked 2-acrylamido-2-methylpropanesulfonic acid homopolymers and crosslinked or non-crosslinked acrylamide copolymers thereof, ammonium acrylate homopolymers, or copolymers of ammonium acrylate and of acrylamide, alone or as mixtures.

**14.** Composition according to any one of Claims 5 to 13, which comprises one or more thickening organic polymers with sugar units, in particular derived from the following sugars: glucose; galactose; arabinose; rhamnose; mannose; xylose; fucose; anhydrogalactose; galacturonic acid; glucuronic acid; mannuronic acid; galactose sulfate; anhydrogalactose sulfate and fructose, more particularly galactose and anhydrogalactose, preferably the thickening organic polymer(s) with sugar units are chosen from agar.

**15.** Composition according to any one of Claims 5 to 14, which comprises one or more thickening organic polymers in a content ranging from 0.01% to 10% by weight, more preferentially from 0.1% to 5% by weight relative to the total weight of the composition.

**16.** Composition according to any one of Claims 5 to 15, which comprises one or more fatty substances as defined in the claims in Claims 10 or 11, and one or more surfactants as defined in any one of Claims 6 to 9, the fatty substance/surfactant(s) weight ratio of which is inclusively between 5 and 20, preferably between 8 and 15, even more preferentially between 10 and 13, such as 11.8.

**17.** Composition according to any one of Claims 5 to 16, which comprises one or more fatty substances as defined in the claims in Claims 10 or 11, and one or more surfactants as defined in any one of Claims 6 to 9, and one or more thickening organic polymers as defined in any one of Claims 12 to 15, the weight ratio of the fatty substance/sum of surfactant(s) and polymer(s) [surfactant(s) + polymer] of which is inclusively between 0.8 and 10, particularly between 1 and 5, more particularly between 1.5 and 2.5, such as 1.9.

**18.** Composition according to any one of Claims 5 to 17, which comprises an aqueous phase and an organic or oily phase, preferably the composition of the invention is a direct emulsion of the oil-in-water (O/W) type.

**19.** Composition according to any one of Claims 5 and 10 to 15, which is aqueous and does not comprise surfactants.

**20.** Composition according to any one of Claims 5 to 19, which comprises one or more organic solvents, in particular chosen from a) $C_2$-$C_6$ alkanols, such as ethanol and isopropanol; b) polyols miscible with water at ambient temperature (25°C) chosen in particular from polyols having in particular from 2 to 10 carbon atoms, preferably having from 2 to 6 carbon atoms, such as glycerol, propylene glycol, 1,3-propanediol, butylene glycol, pentylene glycol, hexylene glycol, dipropylene glycol, diethylene glycol or diglycerol; c) polyol ethers, such as 2-butoxyethanol, propylene glycol monomethyl ether, diethylene glycol monoethyl ether and diethylene glycol monomethyl ether; and also d) aromatic alcohols, such as benzyl alcohol or phenoxyethanol, and mixtures thereof, preferably the composition comprises one or more polyols in particular chosen from polyols having in particular from 2 to 10 carbon atoms, preferably having from 2 to 6 carbon atoms, such as glycerol.

**21.** Composition according to any one of Claims 5 to 20, which comprises one or more organic solvents present in amounts of inclusively between 0.1% and 40% by weight approximately relative to the total weight of the composition, more preferentially between 1% and 20% by weight approximately and even more particularly inclusively between 5% and 10% by weight relative to the total weight of the composition.

**22.** Nontherapeutic cosmetic treatment process for caring for and/or making up and/or cleansing keratin materials, comprising the application to said keratin materials of a composition according to any one of Claims 5 to 21.

**23.** Process for preserving a composition comprising a physiologically acceptable medium, in particular a cosmetic or dermatological composition, **characterized in that** it consists in incorporating into said composition an antimicrobial mixture as defined in one of Claims 1 to 4 or a composition as defined in any one of Claims 5 to 21.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2011039445 A
- US 4131576 A
- EP 503853 A
- FR 2416723
- US 2798053 A
- US 2923692 A
- US 3915921 A
- US 4509949 A
- EP 0173109 A
- WO 0031154 A
- EP 750899 A
- US 5089578 A
- WO 0068282 A
- WO 9844012 A
- RE 205146
- US 5156911 A
- US 5736125 A
- WO 0119333 A
- US 5519063 A
- EP 0550745 A
- US 5783657 A
- US 3645705 A
- US 3148125 A
- US 5500209 A
- FR 2281162
- EP 0497144 A
- WO 9842298 A
- US 6225690 B
- US 6174968 B
- US 6225390 B
- US 5468477 A
- US 5725882 A


### Littérature non-brevet citée dans la description


- **MARTIN M. RIEGER**. Cosmetics. *Kirk Othmer Encyclopedia*, 04 December 2000, https://doi.org/10.1002/0471238961.0315191318090507.a01
- Ullmann's Encyclopedia of Industrial Chemistry. **G. SCHNEIDER et al.** Skin Cosmetics. Wiley-VCH Verlag GmbH & Co. KGaA, 2012, vol. 33, 221
- **WALTER NOLL**. Chemistry and Technology of Silicones. Academie Press, 1968
- Volatile Silicone fluids for cosmetics. Cosmetics and Toiletries. TODD & BYERS, vol. 91, 27-32
- Fats and Fatty Oils. **A. THOMAS**. Ullmann's Encyclopedia of Industrial Chemistry. 15 June 2000
- **YOTARO MORISHIMA**. Self-assembling amphiphilic polyelectrolytes and their nanostructures - Chinese. *Journal of Polymer Science*, 2000, vol. 18, 323-336
- **YOTARO MORISHIMA**. Miscelle formation of random copolymers of sodium 2-(acrylamido)-2-methylpropanesulfonate and a non-ionic surfactant macromonomer in water as studied by fluorescence and dynamic light scattering. *Macromolecules*, 2000, vol. 33, 3694-3704
- **YOTARO MORISHIMA**. Solution properties of miscelle networks formed by non-ionic moieties covalently bound to an polyelectrolyte : salt effects on rheological behavior. *Langmuir*, 2000, vol. 16, 5324-5332
- **YOTARO MORISHIMA**. Stimuli responsive amphiphilic copolymers of sodium 2-(acrylamido)-2-methylpropanesulfonate and associative macromonomers. *Polym. Preprint, Div. Polym. Chem*, 1999, vol. 40 (2), 220-221
- **G. FONNUM** ; **J. BAKKE** ; **FK. HANSEN**. *Colloid Polym. Sci*, 1993, vol. 271, 380-389
- **S. NOJIMA**. Melting behavior of poly(δ-caprolactone)-block-polybutadiène copolymers. *Macromolécules*, 1999, vol. 32, 3727-3734
- **B. BOUTEVIN et al.** Study of morphological and mechanical properties of PP/PBT. *Polymer Bulletin*, 1995, vol. 34, 117-123
- **P. RANGARAJAN et al.** Morphology of semi-crystalline block copolymers of ethylene-(ethylene-alt-propylene). *Macromolecules*, 1993, vol. 26, 4640-4645
- **P. RICHTER et al.** Polymer agregates with crystalline cores : the system poly(ethylene)- poly(ethylene-propylene. *Macromolécules*, 1997, vol. 30 (25), 1053-1068
- **I.W. HAMLEY**. Cristallization in block copolymers. *Advances in Polymer Science*, 1999, vol. 148, 113-137
- Fungal Contaminants in drinking water regulation ?,A tale of ecology, exposure, purification and clinical relevance. *Int. J. Environ. Res. Public Health*, 2017, vol. 14, 636

- **F.C. KULL** ; **P.C. EISMAN** ; **H.D. SYLWESTROWKA** ; **R.L. MAYER**. *Applied Microbiology*, 1961, vol. 9, 538-541